# EUROPÄISCHE PATENTANMELDUNG

(11) **EP 4 169 577 A1**
(43) Veröffentlichungstag der Anmeldung: **26.04.2023**
(21) Anmeldenummer: 21203495.3
(22) Anmeldetag: 19.10.2021
(51) Int. Cl.: A61P 35/00, A61K 31/688, C07F 9/12, C07F 9/6558, C07F 9/6561, C07K 1/00

(54) **4-AMINOPHENYLPHOSPHORYLCHOLIN-VERBINDUNGEN ZUR BLOCKADE VON C-REAKTIVEM PROTEIN**

(71) Anmelder: Pentracor GmbH, 16761 Hennigsdorf (DE)
(72) Erfinder: Kunze, Rudolf, 17268 Flieth-Stegelitz (DE)
(74) Vertreter: Arth, Hans-Lothar

(57) **Zusammenfassung**

Die vorliegende Erfindung betrifft Verbindungen der allgemeinen Formel (**I**), die an C-reaktives Protein binden und/oder neutralisieren. Besonders nützlich sind die erfindungsgemäßen Verbindungen zur Behandlung und/oder Prävention von akuten oder chronischen Erkrankungen, die mit einem erhöhten CRP-Spiegel assoziiert und/oder dadurch verursacht werden.

## Beschreibung

Die vorliegende Erfindung betrifft Verbindungen der allgemeinen Formel (**I**), die an C-reaktives Protein binden und/oder neutralisieren. Besonders nützlich sind die erfindungsgemäßen Verbindungen zur Behandlung und/oder Prävention von akuten oder chronischen Erkrankungen, die mit einem erhöhten CRP-Spiegel assoziiert und/oder dadurch verursacht werden.

In der Humanmedizin ist das C-reaktive Protein (CRP) seit vielen Jahren als Akute Phase Protein bekannt. Insbesondere bei Entzündungen unterschiedlichster Ursache werden erhöhte CRP-Blutspiegel beobachtet. CRP gehört damit zu den wichtigsten Indikatoren von inflammatorischen Krankheitsprozessen. Die Bildung von CRP findet in der Leber statt. Seine Synthese wird durch eine Verletzung und/oder eine Infektion Stunden danach und zeitlich limitiert ausgelöst. Die CRP-Bildung hängt von der Stimulation durch proinflammatorische Zytokine ab, insbes. durch Interleukin-6.

CRP ist ein phylogenetisch altes Molekül und weit verbreitet im Tierreich. Es wurde 1930 zuerst im Blut von Personen mit akuten bakteriellen Infektionen entdeckt. Die physiologisch dominante Form ist ein Pentamer aus identischen Untereinheiten, die nicht kovalent verknüpft sind. Mit einem Molekulargewicht von ca. 125.000 Da gehört es zu den größeren Plasmamolekülen. Konzentrationen von 0,2-3 mg/L Blut werden als Normbereich angesehen.

Im lokalen Mikromilieu, in Gegenwart von niedrigem pH-Wert und oxidativen Stressspezies (ROX), erfährt CRP eine Konformationsänderung, die die Komplementbindung (C1q) ermöglicht, ebenso wie die Dissoziation der pentameren Form (pCRP) in seine Monomere (mCRP). Beides ermöglicht bzw. verstärkt die lokalen Entzündungsprozesse. Die Dissoziation des Pentamers ist dabei ein örtlich begrenztes Geschehen, welches durch Lyso-Phosphocholin noch befördert sein soll. Das monomere CRP soll dabei eine besondere Rolle spielen bei der Zerstörung der Blut-Retina-Barriere (Mollins et al. Front. Immunol.2018, 9:808). Mäßig erhöhte pCRP-Spiegel stehen den Autoren nach dabei im Gleichgewicht zu den lokal pathologisch aktiveren mCRP. In einem Tiermodell (Ratte, Ischämiemodell) wurden von Braig et al. (Nat Commun; 2017, 8: 14188) und Thiele et al. (Front Immunol; 2018, 9: art.6) gezeigt, dass in Kapillargefäßen von Muskelgewebe das Leukozytenrolling und die -Adhärenz durch mCRP erhöht wird, nicht aber durch pCRP. Wahrscheinlich wird die lokale molekulare Entzündungskaskade mit der Bildung von mCRP verstärkt. Die Autoren gehen davon aus, dass die Konformationsänderung von CRP der Schlüssel im Verständnis von vaskulären inflammatorischen Pathomechanismen bei Krankheiten ist, deren Auslöser Gefäßverengungen oder Verschlüsse sind (z.B. beim Herzinfarkt, Schlaganfall).

CRP ist eines der wenigen Plasmamoleküle, deren Konzentration im Blut mehr als hundertfach ansteigen kann. Dies wird ausgelöst durch Gefäßverschlüsse wie bei Herzinfarkt oder Schlaganfall, aber auch durch eine akute Infektion, Sepsis, Brandverletzung, ein schweres Trauma, eine akute Pankreatitis oder Operationen. Dabei steigt die CRP-Konzentration nach einem der o.g. Ereignisse mit einer Verzögerung von mehreren Stunden an. Um ca. 48 Stunden wird z.B. nach einem Herzinfarkt ein Peak beobachtet, während in den Tagen danach der CRP-Spiegel kontinuierlich sinkt. Die individuelle CRP-Responderrate, Ausmaß und Dauer sind sehr unterschiedlich.

Unabhängig voneinander wiesen die Forschungsgruppen um Lucchesi (Barrett et al., J Pharmacol Exp Ther 2002;303(3):1007-1013) und Pepys (Griselli et al, J Exp Med 1999;190(12):1733-1740; Gill et al., J Cereb Blood Flow Metab 2004;24(11):1214-1218) bei Kaninchen bzw. Ratten eine proödematöse und pronekrotische Wirkung von CRP bei Herzinfarkt und Schlaganfall nach. Aus evolutionärer Sicht ist neben einer gewissen antibakteriellen Wirkung die Funktion von CRP die Markierung von nekrotischen und pronekrotischen Zellen, um sie dann mit Hilfe von Komplementproteinen und Phagozyten zu entsorgen und die Gewebsregeneration einzuleiten. Für die Heilung äußerer Wunden ist dieser Mechanismus unabdingbar, für die Heilung innerer, aseptischer Wunden wie Herzinfarkt oder Schlaganfall hingegen sind überschießende CRP-Konzentrationen möglicherweise kontraproduktiv, mit anderen Worten: Auf diese Art von Verletzung bzw. Wunden ist die Evolution nicht eingestellt.

Die molekularen Mechanismen der proinflammatorischen Wirkung von CRP an der Zelloberfläche sind in groben Zügen bekannt. Im Falle einer lokalen Verletzung folgt dort eine akute Entzündung. Es können auf zellulärer Ebene vorübergehend drei Zellpopulationen entstehen: vitale, reversibel defekte und irreversibel defekte Zellen. Die Übergänge zwischen den beiden letzteren sind fließend. CRP ist ein Trigger des Zelluntergangs bzw. der Nekrose, und es hängt wahrscheinlich von der CRP-Menge vor Ort ab, wie hoch der Anteil an reversibel geschädigten Zellen ist, der den "point of no return" erreicht.

Der natürliche Ligand von CRP, das Lyso-Phosphatidylcholin (LPC), ist bei vitalen Zellen als Bestandteil der Zellmembran praktisch nicht vorhanden. Kommt es indes zu einer Zellschädigung, wird LPC durch eine spezielle Phospholipase, ein weiteres akute-Phase-Protein, an der Zelloberfläche erzeugt. Es handelt sich dabei um die sekretorische Phospholipase A2 Typ IIa (sPLA2 IIa). Je mehr CRP vorhanden ist, desto mehr CRP-Moleküle binden an die neu entstehenden LPC-Moleküle. Zellen in einem Infarktareal sind meist schlecht mit Sauerstoff und Nährstoffen versorgt und schalten ihren Stoffwechsel daher von der Atmungskette auf die Glykolyse um, was zu einer Verarmung an Energieäquivalenten führt. Ohne diese energetische Einschränkung würde das neu entstehende LPC sofort wieder repariert werden.

Mit der Bindung an die pronekrotische Zelle bzw. an LPC erfährt CRP eine Konformationsänderung, und es wird die Entzündungskaskade in Gang gesetzt, die nun die Bindung des Komplementproteins C1q an CRP ermöglicht. Daraufhin läuft die Komplementkaskade bis C4 und Faktor H ab. Die Folge ist die Invasion von Monozyten und die Induktion von proinflammatorischen Zytokinen, die ihrerseits in der Leber die Synthese von CRP ankurbeln. Das "Abräumen" von toten bzw. nicht mehr voll funktionsfähigen Zellen, z.B. durch Phagozyten, ist ein essentieller Mechanismus für die Einleitung der Gewebsregeneration und Wundheilung.

Der Normalwert für CRP im Blut von Menschen variiert von Person zu Person, liegt im Median bei ungefähr 0,8 mg CRP pro Liter Blut, kann aber im Fall von akuten oder chronischen Entzündungsreaktionen (z.B. bakterielle Infektionen, Atherosklerose, nach einem Herzinfarkt) auf deutlich über 100 mg CRP pro Liter Blut steigen. Da die Halbwertszeit von CRP im Blut (ca. 19 Stunden) konstant und weitgehend unabhängig vom gesundheitlichen Zustand des Patienten ist, ist allein die Syntheserate von CRP für die Regulation des CRP-Spiegels im Blut verantwortlich (Pepys & Hirschfield, J. Clin. Invest., 2003, 111: 1805-1812). Die stark erhöhte Synthese an CRP bei akuten pathologischen Konditionen stellt folglich besondere Anforderungen an therapeutische Ansätze zur CRP-Entfernung von Patienten (Risikopatienten oder Akut-Patienten), da eine erhebliche Menge an CRP entfernt werden muss, um den CRP-Spiegel im Blut auf Normalwerte zu senken. Die Schäden am Herzen nach einem Herzinfarkt oder am Gehirn nach einem Schlaganfall werden durch CRP und nachfolgende Komplementwirkung noch vergrößert.

Im Wesentlichen sind drei Wege zur Aufhebung der Wirkung von CRP bekannt:
- die Hemmung der CRP-Synthese
- die Eliminierung von CRP aus dem Blutkreislauf
- die pharmakologische Blockade von CRP

Eine effektive Hemmung der CRP-Synthese ist mit Hilfe von Antisense-Oligonukleotiden möglich, welche die CRP-Synthese in der Leber hemmen (Noveck et al., J Am Heart Assoc. 2014; doi.org/10.1161/JAHA.114001084). Gezeigt wurde dies an gesunden Probanden, die nach einer Injektion von Endotoxin hohe Plasmaspiegel an CRP aufwiesen. Allerdings wurde der Wirkstoff ISIS-CRPRx in 6 Dosen über 22 Tage vorab verabreicht. Offensichtlich ist das Oligonukleotid nicht zur Absenkung akuter CRP-Konzentrationen geeignet, wie sie aber bei Herzinfarkt oder Schlaganfall häufig auftreten.

Eine Elimination des CRP aus dem Blutkreislauf bzw. Blutplasma gelingt mittels einer selektiven Apherese durch Verwendung eines CRP-Adsorbers. Die Praktikabilität und Effizienz eines CRP-Apheresesystem bei akuten Entzündungen wurde in einem in einem Infarktmodell am Schwein gezeigt (Sheriff et al., Journal of Clinical Apheresis 2015; 30: 15-21. doi.org/10.1002/jca.21344). Mittels eines CRP-Adsorbers wurde nach Auslösung des Infarktes die CRP-Menge im Blut der Tiere abgesenkt. Die anschließende kardiologische Untersuchung im MRT und der Histologie belegte ein deutlich kleineres Infarktareal und weniger Narbengewebe in der Behandlungsgruppe im Vergleich zu den Kontrolltieren.

Bisherige Befunde einer Pilotstudie an Patienten mit Herzinfarkt bestätigten die positive Wirkung der CRP-Apherese (Ries et al. C-reactive protein apheresis as antiinflammatory therapy in acute myocardial infarction: Results of the CAMI-1 study. Front Cardiovasc Med 2021; 8:591741). Das Infarktareal ist bei Patienten mit CRP-Apherese signifikant kleiner, während die linksventrikuläre Ejektionsfraktion (LVEF) sich signifikant erhöht.

Mit der Absenkung der CRP-Konzentration im Blut wird demnach wahrscheinlich die Menge an CRP an ischämischen bzw. pronekrotischen Zellen (bzw. in deren unmittelbarer Nähe) z.B. eines Infarktareals reduziert. In der Folge stehen weniger Bindungsplätze für Komplementproteine bereit. Dem nur vorgeschädigten, aber reversiblen Teil des betroffenen inflammatorischen Gewebes gelingt die Regeneration. Ob dieser regenerative Prozess, bedingt durch die Absenkung der CRP-Konzentration, bei anderen Geweben als Muskelzellen ebenso stattfindet, ist unbekannt, aber wahrscheinlich.

Der spezifische CRP-Adsorber ist als Medizinprodukt prinzipiell dort einsetzbar, wo im Zuge einer vorzugsweise akuten Entzündung oder Erkrankung mit hohen CRP-Spiegeln die Elimination von CRP von therapeutischem und klinischem Nutzen ist. Das Verfahren ist auf Grund der medizintechnischen Voraussetzungen allerdings nicht immer dort anwendbar, wo die Absenkung von funktionell aktivem CRP bzw. CRP-Blutspiegeln gewünscht ist. Die Anwendung extrakorporaler Aphereseverfahren wie CRP-Apherese, Immunadsorption oder Lipidapherese bedarf eines erheblichen medizintechnischen Aufwandes. Hierin liegt eine gewisse technische Limitation in der Praktikabilität des Verfahrens, so auch der CRP-Apherese.

Die pharmakologische Blockade von CRP ist die dritte therapeutische Option zur Eindämmung der Wirkung von CRP auf vorgeschädigte Zellen oder Gewebe, wie es von Herzinfarkt oder Schlaganfall bekannt ist. Der Wirkstoff 1,6-Bis(phosphocholin)-hexan blockiert CRP *in vivo.* Mittels des sich aus dem natürlichen Liganden Phosphocholin ableitenden Wirkstoffs gelang in einem Experiment an Ratten der Nachweis, dass eine Blockade von CRP zu einem verringerten Volumen des künstlich erzeugten Herzinfarktes führt (Pepys et al., Nature 2006; 440(7088): 1217-1221). Allerdings wurde der CRP-Blocker für seine Wirksamkeit bereits vor dem ausgelösten Herzinfarkt verabreicht und nicht unmittelbar danach. Für die Akutbehandlung eines Infarktes sollte der Wirkstoff seine Wirkung sofort oder zumindest in wenigen Minuten nach dem Infarkt entfalten. Ein Wirkstoff/Medikament, welcher/welches direkt CRP als therapeutisches Target hat, ist derzeit nicht verfügbar.

Aufgabe der vorliegenden Erfindung ist daher die Bereitstellung eines Medikaments zur Bindung und/oder Neutralisation von CRP *in vivo,* insbes. im Blutkreislauf, insbesondere zur Behandlung und/oder Prävention von akuten oder chronischen Erkrankungen, die durch einen erhöhten CRP-Spiegel assoziiert sind und/oder verursacht werden.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Der Erfinder hat überraschenderweise gefunden, dass eine Verbindung der allgemeinen Formel (**I**) CRP bindet und damit zur Bindung und/oder Neutralisation insbes. von freiem, gelösten CRP im Blut geeignet ist. Damit ist die besagte Verbindung besonders geeignet zur Behandlung und/oder Prävention von akuten oder chronischen Erkrankungen, die mit einem erhöhten CRP-Spiegel assoziiert sind und/oder durch ihn verursacht werden.

### Kurzbeschreibung

Die vorliegende Erfindung betrifft eine Verbindung der allgemeinen Formel (**I**): 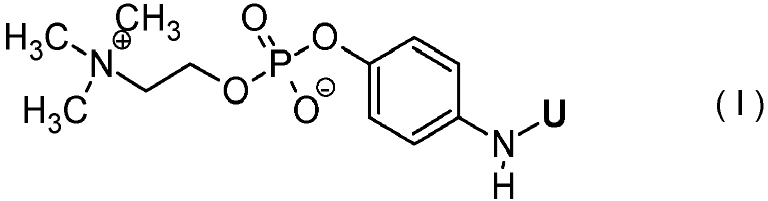 wobei **U** ausgewählt ist aus -**P₃**-**R₃**, -CO-**Z₃**, -**P₃**-**Z₃** oder 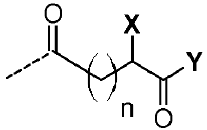 wobei n eine ganze Zahl ausgewählt aus 1, 2 und 3 ist;
**X** -NH₂, -NH-**P₁**-**R₁**, -NH-CO-**Z₁** oder -NH-**P₁**-**Z₁** oder darstellt,
**Y** -OH, -NH₂, -NH-**P₂**-**R₂**, -NH-**Z₂** oder -NH-**P₂**-**Z₂** darstellt,
**P₁**-H, **P₂**-H und **P₃**-H unabhängig voneinander aus 1 - 4 Aminosäuren bestehende Peptidreste darstellen,
**Z₁**, **Z₂** und **Z₃** unabhängig voneinander -(CH₂)ₒ₁-(O-C₂H₄)ₒ₂-O-(CH₂)ₒ₃-**T** darstellen, wobei
   o1 eine ganze Zahl ausgewählt aus 1, 2, 3, 4, 5 und 6 ist
   o2 eine ganze Zahl ausgewählt aus 1, 2, 3, 4, 5, 6, 7, 8, 9 und 10 ist;
   o3 eine ganze Zahl ausgewählt aus 1, 2, 3, 4, 5 und 6 ist;
**T** -H, -OCH₃, -CO-NH₂ oder -NH₂ darstellt;
**R₁**, **R₂** und **R₃** unabhängig voneinander ausgewählt sind aus -H, -CH₃, -CH₂CH₃, -CO-CH₃ und -CO-CH₂CH₃, und Enantiomere, stereoisomere Formen, Mischungen von Enantiomeren, Diastereomere, Mischungen von Diastereomeren, Prodrugs, Hydrate, Solvate, Tautomere und Racemate der vorgenannten Verbindungen und pharmazeutisch verträgliche Salze davon.

Die Peptidreste P₁, P₂ und P₃ bestehen aus α-Aminosäure-Einheiten, die über eine peptidische Amid-Bindung (-CO-NH-) miteinander verbunden sind. Die Aminosäure-Einheiten können von proteinogenen und nichtproteinogenen natürlich vorkommenden wie auch synthetischen Aminosäuren stammen. Sie können L-konfiguriert und D-konfiguriert sein. Innerhalb eines Peptidrests P₁, P₂ oder P₃ können alle Aminosäuren ausschließlich L-konfiguriert (all-L), ausschließlich D-konfiguriert (all-D), oder gemischt L/D-konfiguriert sein. Bei einer gemischten L/D-Konfiguration, können die Konfigurationen statistisch verteilt oder alternierend sein.

Vorzugsweise beginnen die Peptidreste P₁ und P₂ linksseitig an der Carbonylgruppe einer α-Aminosäure-Einheit und enden rechtsseitig mit der terminalen Amino-Gruppe. Vorzugsweise beginnt der Peptidrest P₃ an dem α-Kohlenstoff einer α-Aminosäure-Einheit und ende mit der terminalen Amino-Gruppe der letzten α-Aminosäure-Einheit.

Der Erfinder hat herausgefunden, dass die Verbindung der allgemeinen Formel (**I**) der vorliegenden Erfindung effizient CRP in biologischen Flüssigkeiten bindet und damit besonders zur Behandlung von Erkrankungen geeignet sind, die mit einem erhöhten CRP-Spiegel assoziiert und/oder durch ihn verursacht werden. Insbesondere sind die hierin beschriebenen Verbindungen zur Behandlung und/oder Prävention von Erkrankungen geeignet, die durch ein C-reaktives Protein-Blutspiegel von >>5 mg/L verursacht und/oder damit assoziiert sind.

Ein besonderer Vorteil der erfindungsgemäßen Verbindungen der Formel (**I**) liegt in dessen schneller Verteilung im Blutkreislauf und in den interstitiellen Räumen, wodurch besagte Verbindungen besonders zur akuten Behandlung von Erkrankungen, wie Infarkten oder Schlaganfällen geeignet sind. Dies wird insbesondere durch die verringerte Polarität bzw. gesteigerter Hydrophobizität des Restes **U** erreicht. Eine besonders vorteilhafte Polarität der Verbindung der Formel (**I**) kann mit Resten **U, X** und **Y** erzielt werden, wenn diese D-Aminosäuren enthalten, die sich in den physikochemischen Merkmalen unterscheiden und kombiniert werden.

Weiterhin vorteilhaft sind Verbindungen der allgemeinen Formel (**I**), dessen Reste **U, X** und **Y** Ethylenglycol-Einheiten (PEG) enthalten. Ethylenglycol-Einheiten haben einen positiven Einfluss auf die Polarität bezüglich der Verteilung bzw. Verteilungsgeschwindigkeit im Körper und verzögern zudem den proteolytischen Abbau.

Zudem sind Verbindungen der allgemeinen Formel (**I**) vorteilhaft, dessen Reste **U, X** und **Y** Prolin, Serin und Alanin (PAS) enthalten. Peptide bestehend aus Prolin, Serin und Alanin sind besonders stabil und verzögern so den proteolytischen Abbau.

Die Verbindungen der allgemeinen Formel (**I**) umfassen ein 4-Aminophenylphosphocholin, das unter physiologischen Bedingungen an CRP bindet, sodass die Konzentration an freiem CRP im Blutkreislauf abnimmt. Vorzugsweise binden die Verbindungen der allgemeinen Formel (**I**) der vorliegenden Erfindung monomeres CRP und/oder pentameres CRP.

Ein weiterer Aspekt der vorliegenden Erfindung betrifft eine pharmazeutische Zusammensetzung umfassend die Verbindung der allgemeinen Formel (**I**), einen pharmazeutisch verträglichen Träger, ein Hilfsstoff und/oder ein Verdünnungsmittel.

Ein weiterer Aspekt der vorliegenden Erfindung betrifft die Verwendung der erfindungsgemäßen Verbindung der allgemeinen Formel (**I**) sowie pharmazeutische Zusammensetzungen davon als Medikament zur Bindung und/oder Neutralisation von C-reaktivem Protein im Blut und anderen Körperkompartimenten sowie zur Behandlung von Erkrankungen wie Tumorerkrankungen, Entzündungserkrankungen, Autoimmunerkrankungen, Atemwegserkrankungen, Stoffwechselerkrankungen, Gefäßokklusionen, Herz-Kreislauf-Erkrankungen, postoperativen Zuständen und Infektionserkrankungen.

In einer weiteren Ausführungsform können die hierin beschriebenen erfindungsgemäßen Verbindungen sowie pharmazeutische Zusammensetzungen davon in Kombination mit extrakorporalen Verfahren zur Senkung des CRP-Spiegels, wie Dialyse oder Apherese verwendet werden.

In einer weiteren Ausführungsform können die hierin beschriebenen erfindungsgemäßen Verbindungen sowie pharmazeutische Zusammensetzungen davon in Kombination mit mindestens einen Komplementblocker zur Behandlung von Erkrankungen verwendet werden, die durch einen erhöhten CRP-Spiegel assoziiert und/oder verursacht werden.

### Beschreibung der Erfindung

### Definitionen:

Der Begriff **"physiologische Bedingungen",** wie er hier verwendet wird, bezieht sich auf eine Temperatur, einen pH-Wert, einen osmotischen Druck, eine Osmolalität, oxidativen Stress, eine Elektrolytkonzentration, eine Konzentration eines kleinen organischen Moleküls wie Glucose, Milchsäure, Pyruvat, Nährstoffkomponenten, andere Metaboliten und dergleichen, eine Konzentration eines anderen Moleküls wie Sauerstoff, Karbonat, Phosphat und Kohlendioxid sowie Zelltypen und Nährstoffverfügbarkeit, die am Ort der Verabreichung oder am Gewebe oder Organ am Ort der Wirkung auf ein Subjekt als innerhalb eines normalen Bereichs liegend angesehen werden würden. Vorzugsweise beziehen sich die physiologischen Bedingungen auf die im menschlichen Organismus herrschenden Bedingungen einer Temperatur von 37 °C, einschließlich pathophysiologischen Randbedingungen wie Fieber (Temperatur 37,5 °C - 42 °C), einem pH-Wert von 7,4, einschließlich einer lokalen Variation von 5.0 bis 8.5, etwa bei einer Wunde oder einer Krankheit, und einem osmotischen Druck von ungefähr 300 mosmol/kg.

Der Betriff **"biologische Flüssigkeit",** wie hierin verwendet, bezieht sich auf wässrige Lösungen, die in Säugetieren und vorzugsweise Menschen auftreten und CRP enthalten, wie z.B. Zerebrospinalflüssigkeit, Peritonealflüssigkeit, Pleura-Flüssigkeit, Aszites-Flüssigkeit, Blut, Blutplasma, Leber-Extrakte und Interstitialflüssigkeit.

Der Begriff **"CRP-bindend",** wie er hier verwendet wird, bedeutet, dass die hierin beschriebenen Verbindungen in der Lage sind, über ihre endständige Phosphorylcholin-Gruppe mit C-reaktivem Protein zu reagieren und/oder es zu binden und/oder mit dem CRP einen Komplex zu bilden.

Der Begriff **"Neutralisation",** wie er hier verwendet wird, bezieht sich auf die Verhinderung der proinflammatorischen Wirkung von CRP sowie die Interaktion mit dem humoralen und zellulären Immunsystem. Neutralisation kann somit erreicht werden, indem die Bindung von CRP an Phosphocholin und/oder Phospholipid-Bestandteile zerstörter körpereigener Zellen unterdrückt, oder die Komplementaktivierung verhindert, oder die Bindung an Fresszellen unterbunden wird.

Der Begriff **"Komplementblocker"** oder "Komplementinhibitor", wie er hier verwendet wird, bezieht sich auf Medikamente, die die Aktivität einzelner Komponenten des Komplementsystems oder des gesamten Komplementsystems reduzieren und/oder inhibieren. Komplementblocker oder Komplementinhibitoren binden demnach an Plasmaproteine, wie das C3 und/oder das C5-Plasmaprotein an molekular definierten Stellen und verhindern somit die Komplementaktivierung.

Der Begriff **"postoperativer Zustand",** wie er hier verwendet wird, bezieht sich auf den Zustand eines Patienten nach einem operativen Eingriff (von einem Chirurgen durchgeführte, invasive Intervention in den Körper des Patienten) einschließlich Gefäßverengungen, thromboembolische Erkrankungen und Verletzungsfolgen der Muskulatur, des Bewegungsapparates und Zustand nach Organtransplantation.

Der Begriff "**Körperkompartiment**", wie er hier verwendet wird, bezieht sich auf Teilbereiche des menschlichen Körpers, wie Extrazellulärraum, Interzellularraum, Organe, Gewebe oder Interstitium. Diese Bereiche müssen nicht notwendig räumlich abgrenzbare Strukturen sein, sondern können auch nur gedanklich konstruiert sein.

Die vorliegende Erfindung betrifft CRP-bindende Verbindung der Formel (**I**): 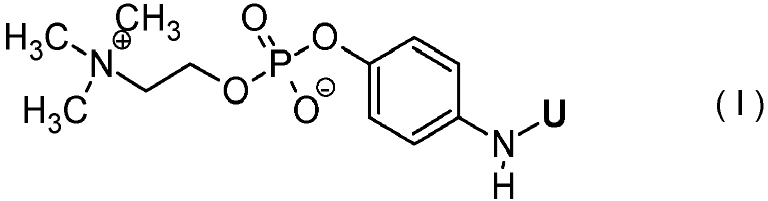 wobei **U** ausgewählt ist aus -**P₃**-**R₃**, -CO-**Z₃**, -**P₃**-**Z₃** oder

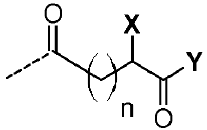

n eine ganze Zahl ausgewählt aus 1, 2 und 3 ist;
**X** -NH₂, -NH-**P₁**-**R₁**, -NH-CO-**Z₁** oder -NH-**P₁**-**Z₁** darstellt,
**Y** -OH, -NH₂, -NH-**P₂**-**R₂**, -NH-**Z₂** oder -NH-**P₂**-**Z₂** darstellt,
**P₁**-H, **P₂**-H und **P₃**-H unabhängig voneinander aus 1 - 4 Aminosäuren bestehende Peptidreste darstellen;
**Z₁**, **Z₂** und **Z₃** unabhängig voneinander -(CH₂)ₒ₁-(O-C₂H₄)ₒ₂-O-(CH₂)ₒ₃-**T** darstellen,

wobei
   o1 eine ganze Zahl ausgewählt aus 1, 2, 3, 4, 5 und 6 ist
   o2 eine ganze Zahl ausgewählt aus 1, 2, 3, 4, 5, 6, 7, 8, 9 und 10 ist;
   o3 eine ganze Zahl ausgewählt aus 1, 2, 3, 4, 5 und 6 ist;
**T** -H, -OCH₃, -CO-NH₂, -NH-CO-CH₃ oder -NH₂ darstellt;
**R₁**, **R₂** und **R₃** unabhängig voneinander ausgewählt sind aus -H, -CH₃, -CH₂CH₃, -CO-CH₃ und -CO-CH₂CH₃,
und Enantiomere, stereoisomere Formen, Mischungen von Enantiomeren, Diastereomere, Mischungen von Diastereomeren, Prodrugs, Hydrate, Solvate, Tautomere und Racemate der vorgenannten Verbindungen und pharmazeutisch verträgliche Salze davon.

Vorzugsweise bestehen die Peptidreste **P₁**, **P₂** und **P₃** nicht aus mehr als einer Aminosäureeinheit. In einer weiteren Ausführungsform wird **U** durch -**P₃**-H dargestellt und **P₃** besteht aus einer Aminosäureeinheit. In einer weiteren Ausführungsform wird **U** durch -**P₃**-H dargestellt und **P₃** besteht aus einer D-konfigurierten Am inosäureeinheit.

Vorzugsweise sind o1 und o3 unabhängig voneinander ausgewählt aus 1 und 2. Vorzugsweise ist o2 eine ganze Zahl ausgewählt aus 1, 2, 3, 4 und 5. In einer bevorzugten Ausführungsform sind o1 und o3 unabhängig voneinander ausgewählt aus 1 und 2 und o2 eine ganze Zahl ausgewählt aus 1, 2, 3, 4 und 5.

Ein weiterer Aspekt der vorliegenden Erfindung betrifft eine Verbindung mit der Struktur der allgemeinen Formel (**II**) 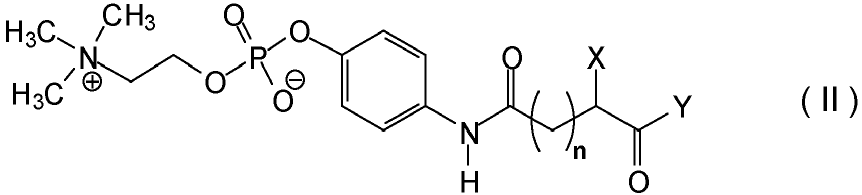 wobei n, **X** und **Y** die hierin angegebenen Bedeutungen haben.

Vorzugsweise ist n eine ganze Zahl ausgewählt aus 2 und 3. Besonders bevorzugt ist, wenn n gleich 2 ist.

In einer bevorzugten Ausführungsform der Verbindung der allgemeinen Formel (**II**) ist **X** eine primäre Aminogruppe und **Y** -OH oder -NH₂. In einer noch bevorzugteren Ausführungsform der Verbindung der allgemeinen Formel (**II**) ist n 2, **Y** -OH und **X** -NH₂.

In einer bevorzugten Ausführungsform der Verbindung der allgemeinen Formel (**II**) sind **X** -NH-CO-**Z₁** und **Y** -OH oder -NH₂, wobei **Z₁** -(CH₂)ₒ₁-(O-C₂H₄)ₒ₂-O-(CH₂)ₒ₃-**T** darstellt und o1, o2, o3 und **T** die hierin angegebenen Bedeutungen besitzen. Vorzugsweise sind o1 und o3 unabhängig voneinander ausgewählt aus 1 und 2 und o2 eine ganze Zahl ausgewählt aus 1, 2, 3, 4 und 5. Noch bevorzugter ist es, wenn o1 1, o2 1, o3 2 und **T** -NH-CO-CH₃ sind.

In einer bevorzugten Ausführungsform der Verbindung der allgemeinen Formel (**II**) sind **X** -NH₂ und **Y** -NH-**Z₂**, wobei **Z₂** -(CH₂)ₒ₁-(O-C₂H₄)ₒ₂-O-(CH₂)ₒ₃-**T** darstellt und o1, o2, o3 und **T** die hierin angegebenen Bedeutungen besitzen. Vorzugsweise sind o1 und o3 unabhängig voneinander ausgewählt aus 1 und 2 und o2 eine ganze Zahl ausgewählt aus 1, 2, 3, 4 und 5. Noch bevorzugter ist es, wenn o1 2, o2 1, o3 1 **und T** -CO-NH₂ sind.

In einer bevorzugten Ausführungsform der Verbindung der allgemeinen Formel (**II**) sind **X** -NH-CO-**Z₁** und **Y** -NH-**Z₂**, wobei **Z₁** -(CH₂)ₒ₁-(O-C₂H₄)ₒ₂-O-(CH₂)ₒ₃-**T** und **Z₂** -(CH₂)ₒ₄-(O-C₂H₄)ₒ₅-O-(CH₂)ₒ₆-**T₂** darstellen; o1, o2, o3 und **T** die hierin angegebenen Bedeutungen besitzen und
o4 eine ganze Zahl ausgewählt aus 1, 2, 3, 4, 5 und 6 ist
o5 eine ganze Zahl ausgewählt aus 1, 2, 3, 4, 5, 6, 7, 8, 9 und 10 ist;
o6 eine ganze Zahl ausgewählt aus 1, 2, 3, 4, 5 und 6 ist;
**T₂** -H, -OCH₃, -CO-NH₂, -NH-CO-CH₃ oder -NH₂ darstellt. Vorzugsweise sind o1, o3, o4 und o6 unabhängig voneinander ausgewählt aus 1 und 2 und o2 und o5 sind eine ganze Zahl unabhängig voneinander ausgewählt aus 1, 2, 3, 4 und 5. Noch bevorzugter ist es, wenn o1 1, o2 1, o3 2, **T** -NH-CO-CH₃, o4 2, o5 1, o6 1 und **T₂** -CO-NH₂ sind.

Besonders bevorzugt sind Verbindungen der allgemeinen Formel (**II**), wobei
**Y** -OH oder -NH₂ darstellt,
**X** -NH₂ oder -NH-CO-**Z₁** darstellt, wobei
**Z₁** -(CH₂)ₒ₁-(O-C₂H₄)ₒ₂-O-(CH₂)ₒ₃-**T** ist, mit
o1 1 oder 2 ist,
o2 eine ganze Zahl ausgewählt aus 1, 2, 3, 4 und 5 ist;
o3 1 oder 2 ist und
**T** -NH₂ darstellt.

In einer weiteren bevorzugten Ausführungsform der Verbindung der allgemeinen Formel (**II**) ist n gleich 2,
stellt **X** -NH₂ oder -NH-**P₁**-**R₁** dar,
stellt **Y** -OH, -NH₂ oder -NH-**P₂**-**R₂** dar,
stellen **P₁** und **P₂** unabhängig voneinander -AS₁-, -AS₁-AS₂- oder -AS₁-AS₂-AS₃- dar und
   sind AS₁, AS₂ und AS₃ für jedes Vorkommen unabhängig voneinander Aminosäuren der folgenden Struktur: 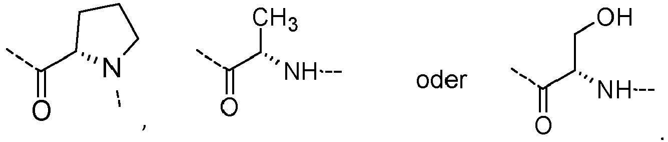 wobei **R₁** und **R**_{**2**'} die hierin angegebenen Bedeutungen aufweisen.

Vorzugsweise sind **P₁** und **P₂** -AS₁-AS₂-AS₃-. Noch bevorzugter ist es, wenn **P₁** -AS₁-AS₂-AS₃- und **P₂** -AS₄-AS₅-AS₆- sind, **R₁** -Ac darstellt und **R₂** -H ist, wobei AS₁ - AS₆ folgende Aminosäuren darstellen:

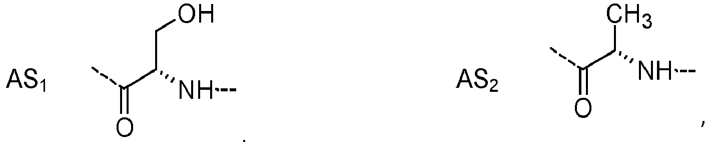

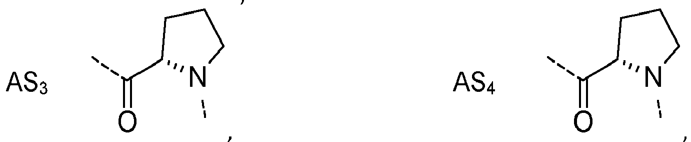

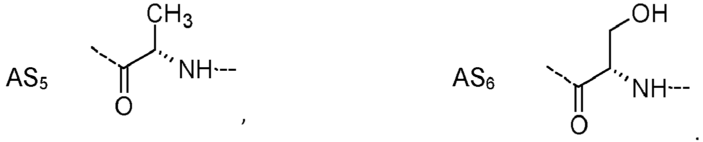

In einer besonders bevorzugten Ausführungsform besitzt die erfindungsgemäße Verbindung die Struktur der allgemeinen Formel (**IIa**) 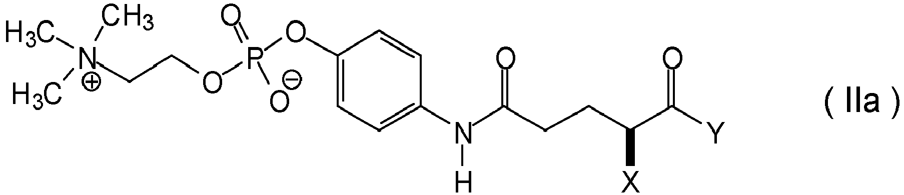 wobei **X** und **Y** die hierin angegebenen Bedeutungen haben.

In einer bevorzugten Ausführungsform der Verbindung der allgemeinen Formel (**IIa**) ist **X** eine primäre Aminogruppe und **Y** -OH oder -NH₂.

In einer bevorzugten Ausführungsform der Verbindung der allgemeinen Formel (**IIa**) sind **X** -NH-CO-**Z₁** und **Y** -OH oder -NH₂, wobei **Z₁** -(CH₂)ₒ₁-(O-C₂H₄)ₒ₂-O-(CH₂)ₒ₃-**T** darstellt und o1, o2, o3 und **T** die hierin angegebenen Bedeutungen besitzen. Vorzugsweise sind o1 und o3 unabhängig voneinander ausgewählt aus 1 und 2 und o2 eine ganze Zahl ausgewählt aus 1, 2, 3, 4 und 5. Noch bevorzugter ist es, wenn o1 1, o2 1, o3 2 und **T** -NH-CO-CH₃ sind.

In einer bevorzugten Ausführungsform der Verbindung der allgemeinen Formel (**IIa**) sind **X** -NH₂ und **Y** -NH-**Z₂**, wobei **Z₂** -(CH₂)ₒ₁-(O-C₂H₄)ₒ₂-O-(CH₂)ₒ₃-**T** darstellt und o1, o2, o3 und **T** die hierin angegebenen Bedeutungen besitzen. Vorzugsweise sind o1 und o3 unabhängig voneinander ausgewählt aus 1 und 2 und o2 eine ganze Zahl ausgewählt aus 1, 2, 3, 4 und 5. Noch bevorzugter ist es, wenn o1 2, o2 1, o3 1 **und T** -CO-NH₂ sind.

In einer bevorzugten Ausführungsform der Verbindung der allgemeinen Formel (**IIa**) sind **X** -NH-CO-**Z₁** und **Y** -NH-**Z₂**, wobei **Z₁** -(CH₂)ₒ₁-(O-C₂H₄)ₒ₂-O-(CH₂)ₒ₃-**T** und **Z₂** -(CH₂)ₒ₄-(O-C₂H₄)ₒ₅-O-(CH₂)ₒ₆-**T₂** darstellen; o1, o2, o3 und **T** die hierin angegebenen Bedeutungen besitzen und
o4 eine ganze Zahl ausgewählt aus 1, 2, 3, 4, 5 und 6 ist
o5 eine ganze Zahl ausgewählt aus 1, 2, 3, 4, 5, 6, 7, 8, 9 und 10 ist;
o6 eine ganze Zahl ausgewählt aus 1, 2, 3, 4, 5 und 6 ist;
**T₂** -H, -OCH₃, -CO-NH₂, -NH-CO-CH₃ oder -NH₂ darstellt. Vorzugsweise sind o1, o3, o4 und o6 unabhängig voneinander ausgewählt aus 1 und 2 und o2 und o5 sind eine ganze Zahl unabhängig voneinander ausgewählt aus 1, 2, 3, 4 und 5. Noch bevorzugter ist es, wenn o1 1, o2 1, o3 2, **T** -NH-CO-CH₃, o4 2, o5 1, o6 1 und **T₂** -CO-NH₂ sind.

Besonders bevorzugt sind Verbindungen der allgemeinen Formel (**IIa**), wobei
**Y** -OH oder -NH₂ darstellt,
**X** -NH₂ oder -NH-CO-**Z₁** darstellt, wobei
**Z₁** -(CH₂)ₒ₁-(O-C₂H₄)ₒ₂-O-(CH₂)ₒ₃-**T** ist, mit
o1 1 oder 2 ist,
o2 eine ganze Zahl ausgewählt aus 1, 2, 3, 4 und 5 ist;
o3 1 oder 2 ist und
**T** -NH₂ darstellt.

In einer weiteren bevorzugten Ausführungsform der Verbindung der allgemeinen Formel (**IIa**)ist n gleich 2 ,
stellt **X** -NH₂ oder -NH-**P₁**-**R₁** dar,
stellt **Y** -OH, -NH₂ oder -NH-**P₂**-**R₂** dar,
stellen **P₁** und **P₂** unabhängig voneinander -AS₁-, -AS₁-AS₂- oder -AS₁-AS₂-AS₃- dar und
   sind AS₁, AS₂ und AS₃ für jedes Vorkommen unabhängig voneinander Aminosäuren der folgenden Struktur: 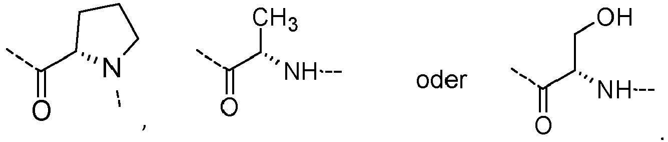 wobei **R₁** und **R**_{**2**'} die hierin angegebenen Bedeutungen aufweisen.

Vorzugsweise sind **P₁** und **P₂** -AS₁-AS₂-AS₃-. Noch bevorzugter ist es, wenn **P₁** -AS₁-AS₂-AS₃- und **P₂** -AS₄-AS₅-AS₆- sind, **R₁** -Ac darstellt und **R₂** -H ist, wobei AS₁ - AS₆ folgende Aminosäuren darstellen:

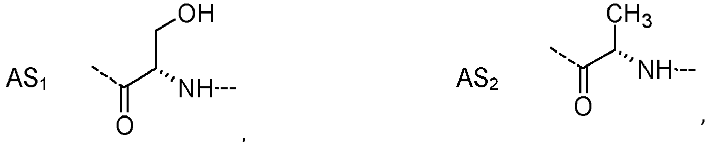

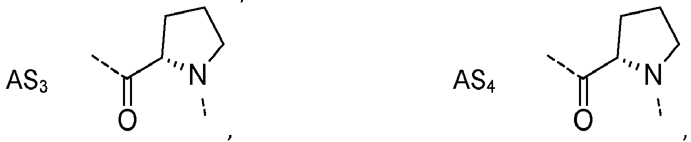

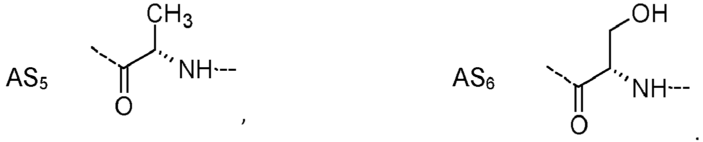

In einer besonders bevorzugten Ausführungsform besitzt die erfindungsgemäße Verbindung die Struktur der allgemeinen Formel (**IIb**) 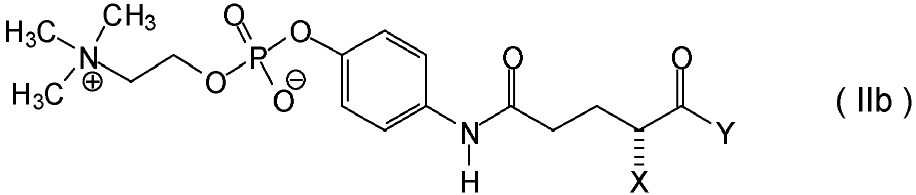 wobei **X** und **Y** die hierin angegebenen Bedeutungen haben.

In einer bevorzugten Ausführungsform der Verbindung der allgemeinen Formel (**IIb**) ist **X** eine primäre Aminogruppe und **Y** -OH oder -NH₂.

In einer bevorzugten Ausführungsform der Verbindung der allgemeinen Formel (**IIb**) sind **X** -NH-CO-**Z₁** und **Y** -OH oder -NH₂, wobei **Z₁** -(CH₂)ₒ₁-(O-C₂H₄)ₒ₂-O-(CH₂)ₒ₃-**T** darstellt und o1, o2, o3 und **T** die hierin angegebenen Bedeutungen besitzen. Vorzugsweise sind o1 und o3 unabhängig voneinander ausgewählt aus 1 und 2 und o2 ist eine ganze Zahl ausgewählt aus 1, 2, 3, 4 und 5. Noch bevorzugter ist es, wenn o1 1, o2 1, o3 2 und **T** -NH-CO-CH₃ sind.

In einer bevorzugten Ausführungsform der Verbindung der allgemeinen Formel (**IIb**) sind **X** -NH₂ und **Y** -NH-**Z₂**, wobei **Z₂** -(CH₂)ₒ₁-(O-C₂H₄)ₒ₂-O-(CH₂)ₒ₃-**T** darstellt und o1, o2, o3 und **T** die hierin angegebenen Bedeutungen besitzen. Vorzugsweise sind o1 und o3 unabhängig voneinander ausgewählt aus 1 und 2 und o2 ist eine ganze Zahl ausgewählt aus 1, 2, 3, 4 und 5. Noch bevorzugter ist es, wenn o1 2, o2 1, o3 1 und **T** -CO-NH₂ sind.

In einer bevorzugten Ausführungsform der Verbindung der allgemeinen Formel (**IIb**) sind **X** -NH-CO-**Z₁** und **Y** -NH-**Z₂**, wobei **Z₁** -(CH₂)ₒ₁-(O-C₂H₄)ₒ₂-O-(CH₂)ₒ₃-**T** und **Z₂** -(CH₂)ₒ₄-(O-C₂H₄)ₒ₅-O-(CH₂)ₒ₆-**T₂** darstellen; o1, o2, o3 und **T** die hierin angegebenen Bedeutungen besitzen und
o4 eine ganze Zahl ausgewählt aus 1, 2, 3, 4, 5 und 6 ist
o5 eine ganze Zahl ausgewählt aus 1, 2, 3, 4, 5, 6, 7, 8, 9 und 10 ist;
o6 eine ganze Zahl ausgewählt aus 1, 2, 3, 4, 5 und 6 ist;
**T₂** -H, -OCH₃, -CO-NH₂, -NH-CO-CH₃ oder -NH₂ darstellt. Vorzugsweise sind o1, o3, o4 und o6 unabhängig voneinander ausgewählt aus 1 und 2 und o2 und o5 sind eine ganze Zahl unabhängig voneinander ausgewählt aus 1, 2, 3, 4 und 5. Noch bevorzugter ist es, wenn o1 1, o2 1, o3 2, **T** -NH-CO-CH₃, o4 2, o5 1, o6 1 und **T₂** -CO-NH₂ sind.

Besonders bevorzugt sind Verbindungen der allgemeinen Formel (**IIb**), wobei
**Y** -OH oder -NH₂ darstellt,
**X** -NH₂ oder -NH-CO-**Z₁** darstellt, wobei
**Z₁** -(CH₂)ₒ₁-(O-C₂H₄)ₒ₂-O-(CH₂)ₒ₃-**T** ist, mit
o1 1 oder 2 ist,
o2 eine ganze Zahl ausgewählt aus 1, 2, 3, 4 und 5 ist;
o3 1 oder 2 ist und
**T** -NH₂ darstellt.

In einer weiteren bevorzugten Ausführungsform der Verbindung der allgemeinen Formel (**IIb**) ist n gleich 2,
stellt **X** -NH₂ oder -NH-**P₁**-**R₁** dar,
stellt **Y** -OH, -NH₂ oder -NH-**P₂**-**R₂** dar,
stellen **P₁** und **P₂** unabhängig voneinander -AS₁-, -AS₁-AS₂- oder
-AS₁-AS₂-AS₃- dar und
   sind AS₁, AS₂ und AS₃ für jedes Vorkommen unabhängig voneinander Aminosäuren der folgenden Struktur: 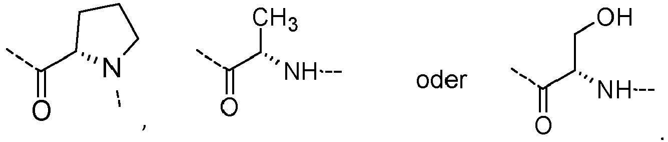 wobei **R₁** und **R**_{**2**'} die hierin angegebenen Bedeutungen aufweisen.

Vorzugsweise sind **P₁** und **P₂** -AS₁-AS₂-AS₃-. Noch bevorzugter ist es, wenn **P₁** -AS₁-AS₂-AS₃- und **P₂** -AS₄-AS₅-AS₆- sind, **R₁** -Ac darstellt und **R₂** -H ist, wobei AS₁ - AS₆ folgende Aminosäuren darstellen:

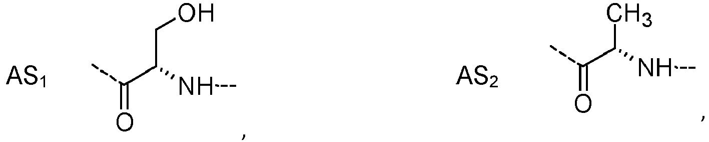

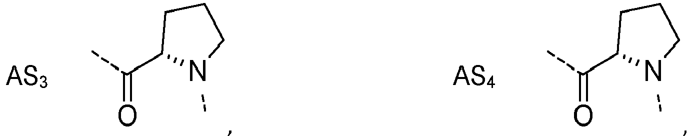

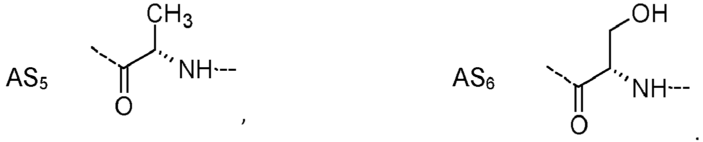

In einer besonders bevorzugten Ausführungsform besitzt die erfindungsgemäße Verbindung die Struktur der allgemeinen Formel (**III**) 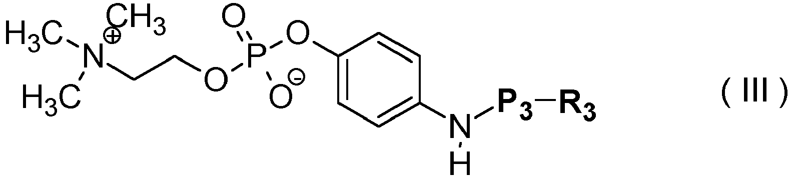 wobei **P₃** ausgewählt ist aus -**Q₁**-, -**Q₁-Q₂**-, -**Q₁-Q₂-Q₃**- und -**Q₁-Q₂-Q₃-Q₄**-;
**Q₁**, **Q₂**, **Q₃** und **Q₄** unabhängig voneinander ausgewählt sind aus 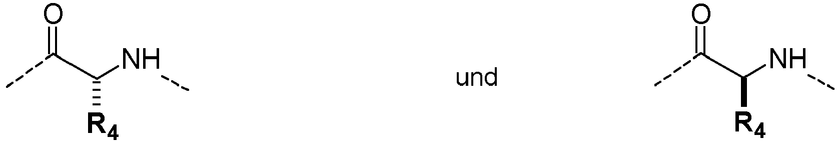 **R₄** unabhängig für jedes Vorkommen -H, -CH₃, -CH₂OH, -CH₂SH, -COOH, -CONH₂, -CH(OH)CH₃, -CH(CH₃)₂, -CH₂-CH(CH₃)₂, -CH(CH₃)-C₂H₅, -CH₂-C₆H₅, -CH₂-C₆H₄-OH, -C₂H₄-S-CH₃, -CH₂-COOH, -CH₂-CONH₂, -C₂H₄-COOH, -C₂H₄-CONH₂, -C₃H₆-NH-C(NH)-NH₂, -C₄H₈-NH₂, 5-imidazolyl oder 2-indolyl darstellt, oder
**R₄** und das benachbarte Stickstoffatom zusammen mit den Atomen, an die sie gebunden sind, einen Pyrrolidin-Ring bilden
und **R₃** die hierin angegebene Bedeutung hat.

Vorzugsweise ist **P₃** -**Q₁**- und **R₃** -H. Bevorzugt ist auch, wenn **R₄** ausgewählt ist aus -H, -CH₂-COOH, -CH₂-CONH₂, -C₂H₄-COOH, -C₂H₄-CONH₂ oder -C₂H₄-S-CH₃.

In einer besonders bevorzugten Ausführungsform besitzt die erfindungsgemäße Verbindung die Struktur der allgemeinen Formel **(IIIa)** 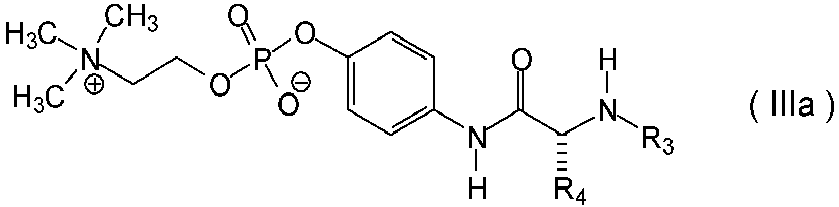 wobei **R₄** unabhängig für jedes Vorkommen -H, -CH₃, -CH₂OH, -CH₂SH, -COOH, -CONH₂, -CH(OH)CH₃, -CH(CH₃)₂, -CH₂-CH(CH₃)₂, -CH(CH₃)-C₂H₅, -CH₂-C₆H₅, -CH₂-C₆H₄-OH, -C₂H₄-S-CH₃, -CH₂-COOH, -CH₂-CONH₂, -C₂H₄-COOH, -C₂H₄-CONH₂, -C₃H₆-NH-C(NH)-NH₂, -C₄H₈-NH₂, 5-imidazolyl oder 2-indolyl darstellt, oder
**R₄** und das benachbarte Stickstoffatom zusammen mit den Atomen, an die sie gebunden sind, einen Pyrrolidin-Ring bilden
und **R₃** die hierin angegebene Bedeutung hat.

Vorzugsweise ist **R₃** -H. Bevorzugt ist auch, wenn **R₄** ausgewählt ist aus -H, -CH₂-COOH, -CH₂-CONH₂, -C₂H₄-COOH, -C₂H₄-CONH₂, -C₂H₄-S-CH oder **R₄** und das benachbarte Stickstoffatom zusammen mit den Atomen, an die sie gebunden sind, einen Pyrrolidin-Ring bilden.

In einer besonders bevorzugten Ausführungsform besitzt die erfindungsgemäße Verbindung die Struktur der allgemeinen Formel (**IIIb**) 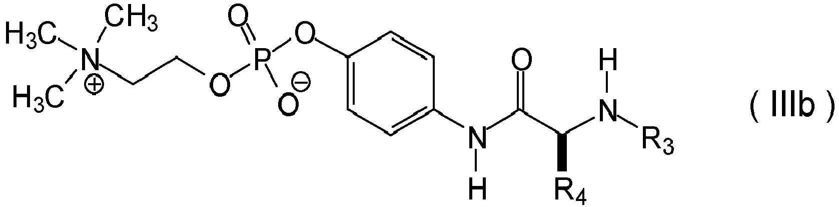 wobei **R₄** unabhängig für jedes Vorkommen -H, -CH₃, -CH₂OH, -CH₂SH, -COOH, -CONH₂, -CH(OH)CH₃, -CH(CH₃)₂, -CH₂-CH(CH₃)₂, -CH(CH₃)-C₂H₅, -CH₂-C₆H₅, -CH₂-C₆H₄-OH, -C₂H₄-S-CH₃, -CH₂-COOH, -CH₂-CONH₂, -C₂H₄-COOH, -C₂H₄-CONH₂, -C₃H₆-NH-C(NH)-NH₂, -C₄H₈-NH₂, 5-imidazolyl oder 2-indolyl darstellt, oder
**R₄** und das benachbarte Stickstoffatom zusammen mit den Atomen, an die sie gebunden sind, einen Pyrrolidin-Ring bilden
und **R₃** die hierin angegebene Bedeutung hat.

Vorzugsweise ist **R₃** -H. Bevorzugt ist auch, wenn **R₄** ausgewählt ist aus -H, -CH₂-COOH, -CH₂-CONH₂, -C₂H₄-COOH, -C₂H₄-CONH₂, -C₂H₄-S-CH oder **R₄** und das benachbarte Stickstoffatom zusammen mit den Atomen, an die sie gebunden sind, einen Pyrrolidin-Ring bilden.

In einer besonders bevorzugten Ausführungsform besitzt die erfindungsgemäße Verbindung die Struktur der allgemeinen Formel (**IIIc**) 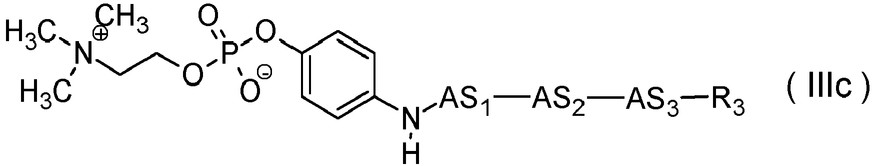 wobei AS₁, AS₂ und AS₃ unabhängig voneinander Aminosäuren der folgenden Struktur darstellen: 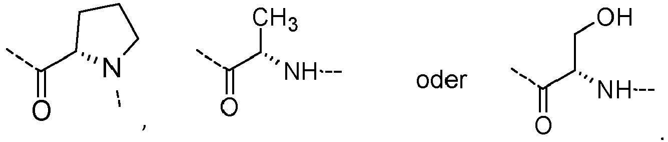 und **R₃** die hierin angegebene Bedeutung hat.

Vorzugsweise ist **R₃** -H.

Bevorzugt ist wenn AS₁ 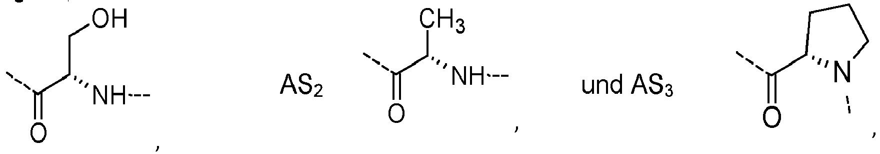 darstellt.

Bevorzugt ist auch, wenn AS₁ 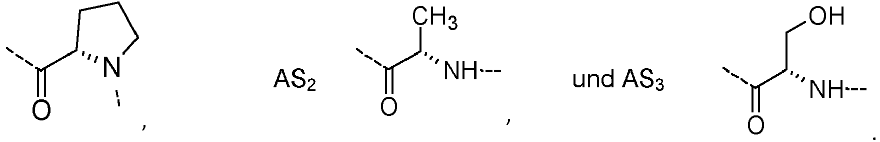 darstellt.

In einer besonders bevorzugten Ausführungsform besitzt die erfindungsgemäße Verbindung die Struktur der allgemeinen Formel (**IV**) 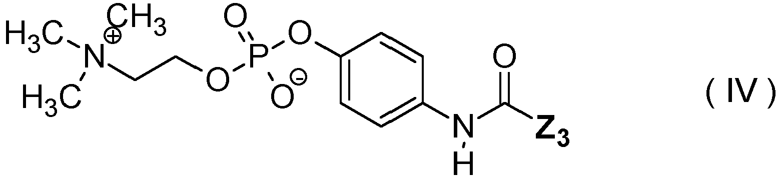 wobei **Z₃**-(CH₂)ₒ₁-(O-C₂H₄)ₒ₂-O-(CH₂)ₒ₃-**T** darstellt,
o1 eine ganze Zahl ausgewählt aus 1, 2, 3, 4, 5 und 6 ist
o2 eine ganze Zahl ausgewählt aus 1, 2, 3, 4, 5, 6, 7, 8, 9 und 10 ist;
o3 eine ganze Zahl ausgewählt aus 1, 2, 3, 4, 5 und 6 ist;
**T** -H, -OCH₃, -CO-NH₂, -NH-CO-CH₃ oder -NH₂ darstellt.

Vorzugsweise ist o1 1 oder 2 ,
o2 eine ganze Zahl ausgewählt aus 1, 2, 3, 4 und 5,
o3 1 oder 2, und
**T** -NH₂.

Besonders bevorzugt sind die Verbindungen 1, 3, 4 und 5.

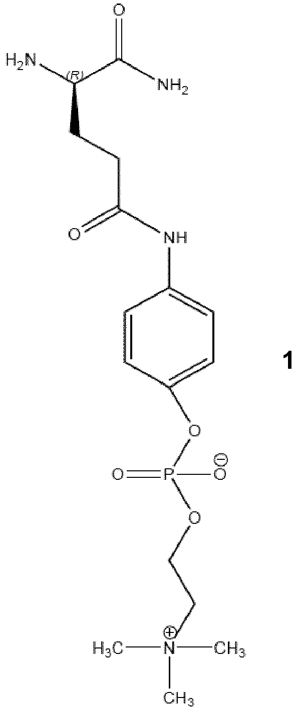

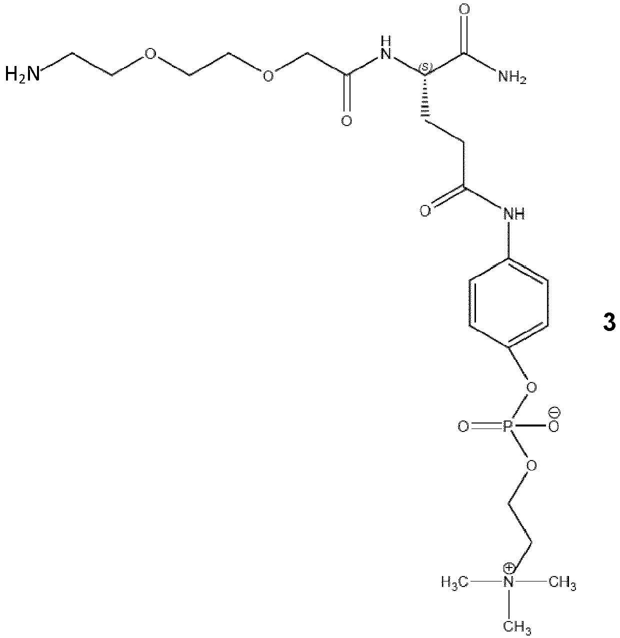

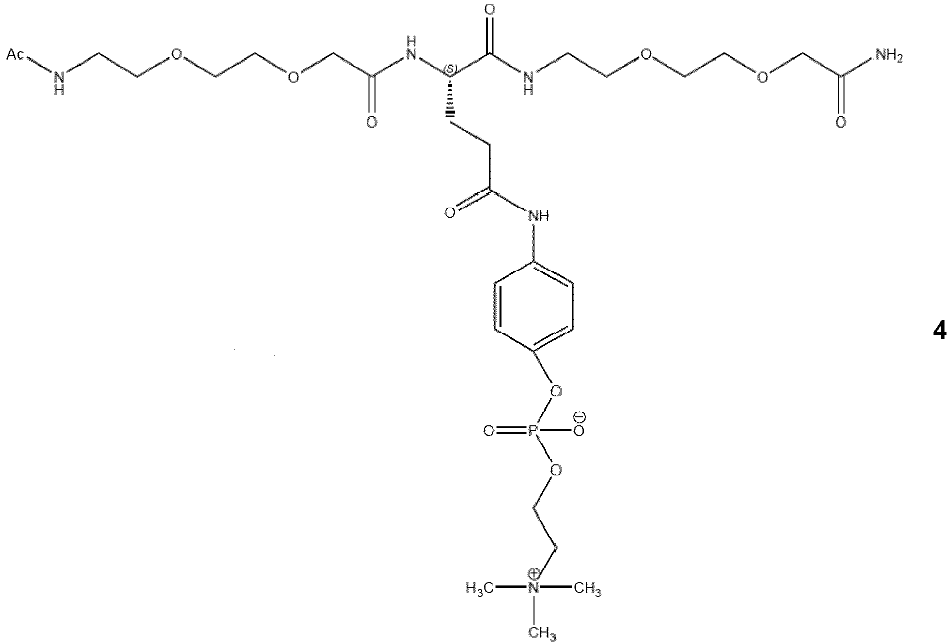

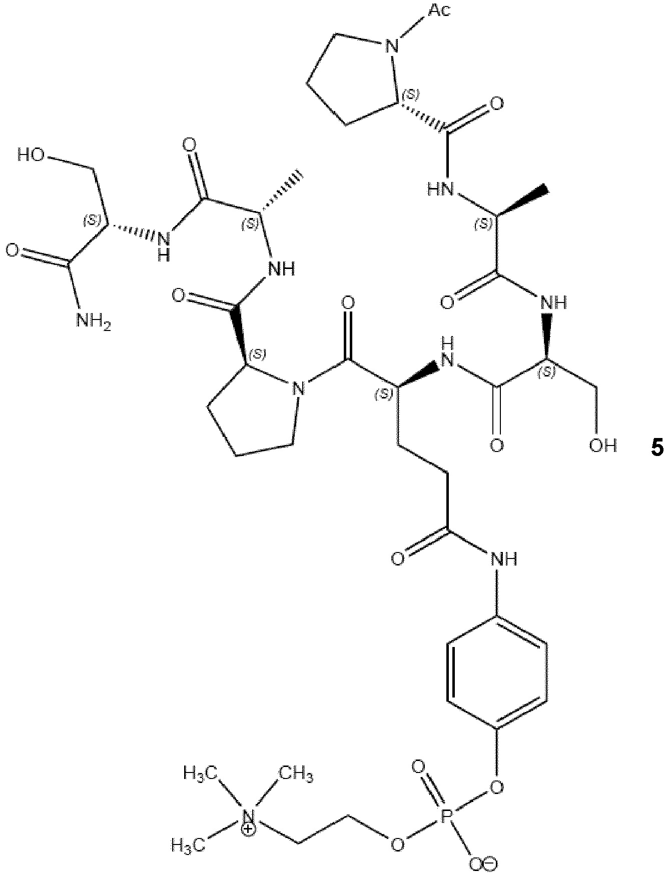

Der Erfinder hat überraschenderweise herausgefunden, dass die Verbindungen der allgemeinen Formeln (**I**), (**II**), (**IIa**), (**IIb**), (**III**), (**IIIa**), (**IIIb**), (**IIIc**) und (**IV**) effizient an CRP binden und dadurch die entzündungsbedingte Zell-, Gewebs- und Gefäßdestruktion reduzieren. Die hierin beschriebenen Verbindungen der allgemeinen Formeln (**I**), (**II**), (**IIa**), (**IIb**), (**III**), (**IIIa**), (**IIIb**), (**IIIc**) und (**IV**) sind in der Lage, so an das CRP zu binden, dass eine anschließende Komplexierung mit dem Komplementprotein C1q nicht mehr oder nur in geringerem stattfindet. Dadurch wird die proödematöse und pronekrotische Wirkung von CRP bei Erkrankungen wie Herzinfarkt und Schlaganfall gestoppt, verringert oder gar ausgesetzt. Letztlich kann so die Ausbreitung von Schäden und Ausbildung von Narben im ischämischen Gewebe gestoppt und eine Infarktnarbe nach Herzinfarkt verringert oder gänzlich verhindert werden.

Die hier beschriebenen Verbindungen der allgemeinen Formeln (**I**), (**II**), (**IIa**), (**IIb**), (**III**), (**IIIa**), (**IIIb**), (**IIIc**) und **(IV)** können aus 4-Aminophenylphosphocholin und Aminosäurebausteinen mit den aus dem Stand der Technik bekannten Methoden zur Synthese von Peptidkonjugaten hergestellt werden. Aminosäuren weisen eine dem Fachmann bekannte Vielzahl an funktionellen Gruppen auf, über die das 4-Aminophenylphosphocholin (APPC) kovalent gebunden werden kann. Vorzugsweise wird das APPC über eine α-Carboxylgruppe oder eine Carboxylgruppe eines Glutamat- oder Aspartat-Restes gebunden. Die Aminosäuren können mit den etablierten Schutzgruppen-Strategien, wie Fmoc- oder Boc-Schutzgruppen eingesetzt werden.

Insbesondere Peptidkupplungsreagenzien wie Carbodiimide (einschließlich Dicyclohexylcarbodiimid (DCC), Diisopropylcarbodiimid (DIC) und 1-Ethyl-3-(3-dimethylaminopropyl)carbodiimid (EDC)), Phosphoniumsalze (einschließlich BOP-Reagenz, PyBOP, PyBrOP und PyOxim), Immoniumsalze (einschließlich BOMI und BDMP), Aminiumsalze (einschließlich HBTU, TBTU, HATU, HCTU und TATU), Uroniumsalze (einschließlich TNTU, TPTU, TOTU, TDBTU, COMU, COMBU, TOMBU und TSTU), Imidazoliumsalze (einschließlich CBMIT, BOI, CIP, CIB und CMBI) oder Carbonyldiimidazol sind für die Bindung des APPC an den peptidischen Rest **U** geeignet.

Der Erfinder hat herausgefunden, dass die hierin beschriebenen Verbindungen der allgemeinen Formeln (**I**), (**II**), (**IIa**), (**IIb**), (**III**), (**IIIa**), (**IIIb**), (**IIIc**) und **(IV)** effizient CRP im Blutkreislauf binden und damit besonders zur Behandlung von Erkrankungen geeignet sind, die durch einen erhöhten CRP-Spiegel assoziiert und/oder verursacht werden. Ein erhöhter CRP-Spiegel bedeutet hierin ein C-reaktives Protein-Blutspiegel von vorzugsweise >5 mg/L, noch bevorzugter von >10 mg/L, noch bevorzugter von >15 mg/L, noch bevorzugter von >20 mg/L, noch bevorzugter von >25 mg/L, noch bevorzugter von >30 mg/L, noch bevorzugter von >50 mg/L, noch bevorzugter von >75 mg/L, noch bevorzugter von >100 mg/L, noch bevorzugter von >150 mg/L und am bevorzugtesten von > 200 mg/L zu einem beliebigen Zeitpunkt der Erkrankung.

Somit liegt ein weiterer Aspekt der vorliegenden Erfindung in der Verwendung der Verbindungen der allgemeinen Formeln (**I**), (**II**), (**IIa**), (**IIb**), (**III**), (**IIIa**), (**IIIb**), (**IIIc**) und **(IV)** in der Behandlung und/oder Prävention von Erkrankungen, die durch einen CRP-Blutspiegel von >20 mg/L verursacht und/oder damit assoziiert sind, wobei **U** -H, -**P₃**-**R₃**, -CO-**Z₃**, -**P₃**-**Z₃** oder

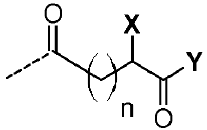

ist und n, **X, Y, P₃, R₃, R₄,** AS₁, AS₂, AS₃ und **Z₃** die hierin angegebenen Bedeutungen haben.

Zu den Erkrankungen, die mit einem erhöhten CRP-Spiegel assoziiert sind und/oder verursacht werden, gehört jedoch nicht Atherosklerose. Bei der Atherosklerose wird regelmäßig kein erhöhter CRP-Spiegel von >20 mg/L im Blut beobachtet.

Somit betrifft die vorliegende Erfindung die Verbindungen der allgemeinen Formel (**I**) zur Verwendung in der Behandlung und/oder Prävention von Erkrankungen, die durch einen C-reaktives Protein-Blutspiegel von >20 mg/L verursacht und/oder assoziiert sind, wobei die Erkrankung nicht Atherosklerose ist wobei **U** -H, -**P₃**-**R₃**, -CO-**Z₃**, -**P₃**-**Z₃** oder 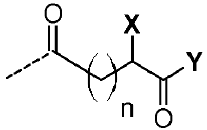 ist und n, **X, Y, P₃, R₃, R₄,** AS₁, AS₂, AS₃ und **Z₃** die hierin angegebenen Bedeutungen haben.

Bei den Erkrankungen, die durch einen erhöhten CRP-Spiegel assoziiert und/oder verursacht werden, handelt es sich vorzugsweise um akute Erkrankungen, bei denen ein erhöhter CRP-Spiegel (wie er hier beschrieben ist) im Patienten innerhalb von 1 bis 5 Tagen beobachtbar ist. Vorzugsweise werden diese Erkrankungen durch einen Anstieg des CRP-Spiegels von >20 mg/L innerhalb von 1 bis 5 Tagen assoziiert und/oder verursacht. Vorzugsweise werden diese Erkrankungen durch einen Anstieg des CRP-Spiegels von >20 mg/L innerhalb von 1 bis 4 Tagen assoziiert und/oder verursacht. Vorzugsweise werden diese Erkrankungen durch einen Anstieg des CRP-Spiegels von >20 mg/L innerhalb von 1 bis 3 Tagen assoziiert und/oder verursacht. Vorzugsweise werden diese Erkrankungen durch einen Anstieg des CRP-Spiegels von >20 mg/L innerhalb von 1 bis 2 Tagen assoziiert und/oder verursacht.

Somit betrifft die vorliegende Erfindung die Verbindungen der allgemeinen Formeln (**I**), (**II**), (**IIa**), (**IIb**), (**III**), **(IIIa),** (**IIIb**), (**IIIc**) und **(IV)** zur Verwendung in der Behandlung und/oder Prävention von Erkrankungen, die durch einen akuten Anstieg des CRP -Blutspiegels von >20 mg/L verursacht und/oder assoziiert sind wobei **U** -H, -**P₃**-**R₃**, -CO-**Z₃**, -**P₃**-**Z₃** oder

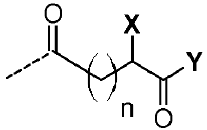

ist

und n, **X, Y, P₃, R₃, R₄,** AS₁, AS₂, AS₃ und **Z₃** die hierin angegebenen Bedeutungen haben.

Zu den Erkrankungen, die mit einem erhöhten CRP-Spiegel assoziiert und/oder dadurch verursacht werden, gehören u.a. Tumorerkrankungen, Entzündungserkrankungen, Stoffwechselerkrankungen, Autoimmunerkrankungen, Atemwegserkrankungen, Herz-Kreislauf-Erkrankungen, postoperative Zustände und Infektionserkrankungen.

Somit betrifft die vorliegende Erfindung die Verbindungen der allgemeinen Formeln (**I**), (**II**), (**IIa**), (**IIb**), (**III**), **(IIIa),** (**IIIb**), (**IIIc**) und **(IV)** zur Verwendung in der Behandlung und/oder Prävention von Erkrankungen ausgewählt aus Tumorerkrankungen, Entzündungserkrankungen, Stoffwechselerkrankungen, Autoimmunerkrankungen, Atemwegserkrankungen, Herz-Kreislauf-Erkrankungen, postoperative Zustände und Infektionserkrankungen wobei **U** -H, -**P₃**-**R₃**, -CO-**Z₃**, -**P₃**-**Z₃** oder 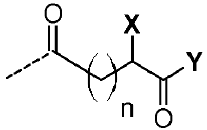 ist und n, **X, Y, P₃, R₃, R₄,** AS₁, AS₂, AS₃ und **Z₃** die hierin angegebenen Bedeutungen haben.

Vorzugsweise handelt es sich bei den Tumorerkrankungen um Erkrankungen, die ausgewählt sind aus Adenokarzinom, choroidales Melanom, akute Leukämie, akustisches Neurinom, ampulläres Karzinom, Analkarzinom, Astrocytom, Basalzellenkarzinom, Bauchspeicheldrüsenkrebs, Desmoid-Tumor, Blasenkrebs, Bronchialkarzinom, Brustkrebs, Burkitt-Lymphom, Korpuskrebs, CUP-Syndrom (Karzinom unbekannten Ursprungs), Darmkrebs, Dünndarmkrebs, Dünndarmtumore, Eierstockkrebs, endometriales Karzinom, Ependymom, epitheliale Krebsformen, Ewing-Tumore, gastrointestinale Tumore, Magenkrebs, Gallenblasenkrebs, Gallenblasenkarzinome, Uteruskrebs, Zervixkrebs, Zervix, Glioblastome, gynäkologische Tumore, Ohr-, Nasen- und Halstumore, hämatologische Neoplasien, Haarzellenleukämie, Harnröhrenkrebs, Hautkrebs, Haut-Hodenkrebs, Hirntumore (Gliome), Hirnmetastasen, Hodenkrebs, Hypophysentumor, Karzinoide, Kaposi-Sarkom, Kehlkopfkrebs, Keimzellentumor, Knochenkrebs, Darmkarzinom, Kopf- und Halstumore (Tumore des Ohren-, Nasen- und Halsbereichs), Kolonkarzinom, Kraniopharyngiome, Mundkrebs (Krebs im Mundbereich und an den Lippen), Krebs des zentralen Nervensystems, Leberkrebs, Lebermetastasen, Leukämie, Augenlidtumor, Lungenkrebs, Lymphknotenkrebs (Hodgkin/Non-Hodgkin), Lymphome, Magenkrebs, malignes Melanom, maligne Neoplasie, maligne Tumore des Gastrointestinaltrakts, Brustkarzinom, Mastdarmkrebs, Medulloblastome, Melanom, Meningiome, Hodgkin-Krankheit, Mykosis fungoides, Myositis, Nasenkrebs, Neurinom, Neuroblastom, Nierenkrebs, Nierenzellkarzinome, non-Hodgkin-Lymphome, Oligodendrogliom, Ösophaguskarzinom, osteolytische Karzinome und osteoplastische Karzinome, Osteosarkome, Ovarialkarzinom, Pankreaskarzinom, Peniskrebs, Plasmocytom, Prostatakrebs, Rachenkrebs, Mastdarmkarzinom, Retinoblastom, Vaginalkrebs, Schilddrüsenkarzinom, Schneeberger-Krankheit, Speiseröhrenkrebs, Spinaliome, T-Zellen-Lymphom (Mykosis fungoides), Thymom, Tubenkarzinom, Augentumore, Harnröhrenkrebs, urologische Tumor, urotheliales Karzinom, Vulvakrebs, Auftreten von Warzen, Weichteiltumore, Weichteilsarkom, Wilmstumor, Zervixkarzinom und Zungenkrebs.

Die vorliegende Erfindung betrifft zudem die Verbindungen der allgemeinen Formeln (**I**), (**II**), (**IIa**), (**IIb**), (**III**), (**IIIa**), (**IIIb**), (**IIIc**) und (**IV**) zur Verwendung in der Behandlung und/oder Prävention von Autoimmunerkrankungen ausgewählt aus Asthma, Diabetes, rheumatische Erkrankungen, AIDS, Abstoßung transplantierter Organe und Gewebe, Rhinitis, chronisch-obstruktive Lungenkrankheiten, Osteoporose, Colitis ulcerosa, Sinusitis, Lupus erythematodes, rekurrierende Infektionen, atopische Dermatitis / Ekzeme und berufsbedingte Allergien, Nahrungsmittelallergien, Arzneimittelallergien, schwere anaphylaktische Reaktionen, Anaphylaxie, Manifestationen allergischer Krankheiten, primäre Immunschwäche, Antikörpermangelzustände, Zell-vermittelte Immunschwäche, schwere kombinierte Immunschwäche, DiGeorge-Syndrom, Hyper-IgE-Syndrom, Wiskott-Aldrich-Syndrom, Ataxia teleangiectasia, Immun-vermittele Krebsformen, Leukozyten-Defekte, Autoimmunkrankheiten, systemischer Lupus erythematodes, rheumatoide Arthritis (RA), Multiple Sklerose (MS), Immun-vermittelter oder Typ 1-Diabetes mellitus, Immun-vermittelte Glomerulonephritis, Sklerodermie, perniziöse Anämie, Alopezie, Pemphigus, Pemphigus vulgaris, Myasthenia gravis, entzündliche Darmerkrankungen, Morbus Crohn, Psoriasis, Autoimmun-Schilddrüsenerkrankungen, Hashimoto-Thyreoditis, Dermatomyositis, Goodpasture-Syndrom, Myasthenia gravis pseudoparalytica, Ophthalmia sympathica, phakogene Uveitis, chronisch-aggressive Hepatitis, primäre biliäre Zirrhose, autoimmunhämolytische Anämie und Morbus Werlhof wobei **U** -H, -**P₃**-**R₃**, -CO-**Z₃**, -**P₃**-**Z₃** oder 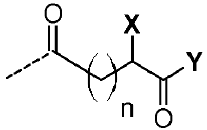 ist und n, **X, Y, P₃, R₃, R₄,** AS₁, AS₂, AS₃ und **Z₃** die hierin angegebenen Bedeutungen haben.

Vorzugsweise werden die Verbindungen der allgemeinen Formeln (**I**), (**II**), (**IIa**), (**IIb**), (**III**), **(IIIa),** (**IIIb**), (**IIIc**) und **(IV)** zur Behandlung und/oder Prävention von Infektionskrankheiten verwendet, die ausgewählt sind aus AIDS, alveoläre Echinokokkose (AHD, Echinokokkose), Amöbiasis *(Entamoeba histolytica*-Infektion), Angiostrongylus-Infektion, Anisakiase, Anthrax, Babesiose (Babesia-Infektion), Balantidium-Infektion (Balantidiose), Baylisascaris-Infektion (Waschbärspulwurm), Bilharzia (Schistosomiasis), *Blastocystis hominis*-Infektion (Blastomykose), Borreliose, Botulismus, Brainerd-Diarrhöe, Brucellose, BSE (Bovine Spongiforme Encephalopathie), Candidiasis, Capillariasis (Capillaria-Infektion), CFS (chronisches Ermüdungssyndrom), Chagas-Krankheit (Amerikanische Trypanosomiasis), Windpocken *(Varicella zoster*-Virus), *Chlamydia pneumoniae-*Infektion, Cholera, chronisches Ermüdungssyndrom, CJD (Creutzfeldt-Jakob-Krankheit), Clonorchiasis (Clonorchis-Infektion), LMC (*Larva migrans cutanea,* Hakenwurm-Infektion), Kokzidioidomykose, Konjunktivitis, Coxsackievirus A16 (Hand-, Maul- und Klauenseuche), Kryptokokkose, Cryptosporidium-Infektion (Kryptosporidiose), Culex-Stechmücke (Vektor des West-Nil-Virus), *Larva migrans cutanea* (LMC), Cyclosporose (Cyclospora-Infektion), Cysticercose (Neurocysticercose), Cytomegalovirus-Infektion, Dengue / Dengue-Fieber, Dipylidium-Infektion (Hunde- und Katzenflohbandwurm), Ebola-Virus hämorrhagisches Fieber, Echinokokkose (Alveoläre Echinokokkose), Encephalitis, *Entamoeba coli*-Infektion, *Entamoeba dispar*-Infektion, *Entamoeba hartmanni*-Infektion, *Entamoeba histolytica*-Infektion (Amöbiasis), *Entamoeba polecki*-Infektion, Enterobiasis (Madenwurm-Infektion), Enterovirus-Infektion (Non-Polio), Epstein-Barr-Virus-Infektion, *Escherichia coli-*Infektion, lebensmittelbedingte Infektion, Maul- und Klauenseuche, fungale Dermatitis, Gastroenteritis, Streptokokken-Erkrankung der Gruppe A, Streptokokken-Erkrankung der Gruppe B, Morbus Hansen (Lepra), Hantavirales Pulmonares Syndrom, Befall mit Kopfläusen (Pedikulose), *Helicobacter pylori-*Infektion, hämatologische Krankheit, Hendra-Virus-Infektion, Hepatitis (HCV, HBV), *Herpes zoster* (Gürtelrose), HIV-Infektion, humane Ehrlichiose, humane Parainfluenza-Virus-Infektion, Influenza, Isosporiose (Isospora-Infektion), Lassa-Fieber, Leishmaniose, Kala-azar (Kala-azar-, Leishmania-Infektion), Lepra, Läuse (Kleiderläuse, Kopfläuse, Filzläuse), Lyme-Krankheit, Malaria, vom Marburg-Virus ausgelöstes hämorrhagisches Fieber, Masern, Meningitis, von Stechmücken übertragene Krankheiten, *Mycobacterium avium*-Komplex (MAC)-Infektion, Naegleria-Infektion, nosokomiale Infektionen, nicht-pathogenene intestinale Amöben-Infektion, Onchozerkose (Flußblindheit), Opistorchose (Opisthorchis-Infektion), Parvovirus-Infektion, Pest, PCP *(Pneumocystis carinii*-Pneumonie), Polio, Q-Fieber, Tollwut, Respiratorische Syncytial-Virus (RSV)-Infektion, rheumatisches Fieber, Rifttal-Fieber, Flußblindheit (Onchozerkose), Rotavirus-Infektion, Spulwurm-Infektion, Salmonellose, *Salmonella enteritidis,* Krätze, Shigellose, Gürtelrose, Schlafkrankheit, Pocken, Streptokokken-Infektion, Bandwurm-Infektion (Taenia-Infektion), Tetanus, toxisches Schocksyndrom, Tuberkulose, Geschwüre (Magengeschwür), Tal-Fieber, *Vibrio parahaemolyticus-*Infektion, *Vibrio vulnificus*-Infektion, virales hämorrhagisches Fieber, Warzen, über das Wasser übertragene Infektionskrankheiten, West-Nil-Virus-Infektion (West-Nil-Encephalitis), Keuchhusten, Gelbfieber, Tuberkulose, Lepra und durch Mykobakterien verursachte Meningitis wobei **U** -H, -**P₃**-**R₃**, -CO-**Z₃**, -**P₃**-**Z₃** oder 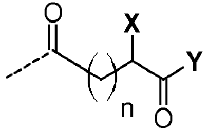 ist und n, **X, Y, P₃, R₃, R₄,** AS₁, AS₂, AS₃ und **Z₃** die hierin angegebenen Bedeutungen haben.

Vorzugsweise betrifft die vorliegende Erfindung die Verwendung der Verbindungen der allgemeinen Formeln (**I**), (**II**), (**IIa**), (**IIb**), (**III**), (**IIIa**), (**IIIb**), (**IIIc**) und **(IV)** zur Behandlung und/oder Prävention von Entzündungserkrankungen ausgewählt aus entzündliche Erkrankungen des zentralen Nervensystems (ZNS), entzündliche rheumatische Erkrankungen, entzündliche Erkrankungen der Blutgefäße, entzündliche Erkrankungen des Mittelohrs, entzündliche Darmerkrankungen, entzündliche Erkrankungen der Haut, entzündliche Erkrankung Uveitis und entzündliche Erkrankungen des Kehlkopfes wobei **U** -H, -**P₃**-**R₃**, -CO-**Z₃**, -**P₃**-**Z₃** oder 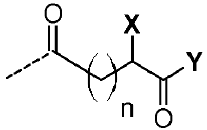 ist und n, **X, Y, P₃, R₃, R₄,** AS₁, AS₂, AS₃ und **Z₃** die hierin angegebenen Bedeutungen haben.

Vorzugsweise betrifft die vorliegende Erfindung die Verwendung der Verbindungen der allgemeinen Formeln (**I**), (**II**), (**IIa**), (**IIb**), (**III**), (**IIIa**), (**IIIb**), (**IIIc**) und (**IV**) zur Behandlung und/oder Prävention von Atemwegserkrankungen ausgewählt aus Asthma Bronchiale, pädiatrisches Asthma, schweres Asthma, akuter Asthma-Anfall, chronische Bronchitis, COPD (chronisch obstruktive pulmonale Erkrankung) und Interstitielle Lungenerkrankungen, wie Pneumonien, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Vorzugsweise betrifft die vorliegende Erfindung die Verwendung der Verbindungen der allgemeinen Formeln (**I**), (**II**), (**IIa**), (**IIb**), (**III**), (**IIIa**), (**IIIb**), (**IIIc**) und (**IV**) zur Behandlung und/oder Prävention von Stoffwechselerkrankungen ausgewählt aus Adrenogenitales Syndrom, Alkaptonurie, Alpha-1-Antitrypsinmangel, Diabetes mellitus (Zuckerkrankheit), Erythropoetische Protoporphyrie (Erkrankung aus der Gruppe der Porphyrien), Galaktosämie, Hypophosphatasie (Rathbuin-Syndrom), Hypothyreose (Schilddrüsenunterfunktion), Ketoazidose, Ketose (Acetonämie, Acetonurie), Lesch-Nyhan-Syndrom (Hyperurikämie-Syndrom oder Hyperurikose), Methylmalonazidurie (MMA), Myoadenylatdeaminase-Mangel (MADD), Morbus Addison (Hypadrenokortizismus), Morbus Conn (Hyperaldosteronismus), Morbus Cushing, Morbus Fabry, Morbus Gaucher, Morbus Hunter (Mukopolysaccharidose Typ II), Mukoviszidose (zystische Fibrose), Phenylketonurie, Porphyrien, Thesaurismose (Speicherkrankheit) und Urikopathie (Gicht) wobei **U** -H, -**P₃**-**R₃**, -CO-**Z₃**, -**P₃**-**Z₃** oder 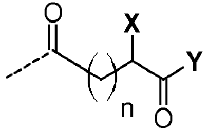 ist und n, **X, Y, P₃, R₃, R₄,** AS₁, AS₂, AS₃ und **Z₃** die hierin angegebenen Bedeutungen haben.

Vorzugsweise betrifft die vorliegende Erfindung die Verwendung der Verbindungen der allgemeinen Formeln (**I**), (**II**), (**IIa**), (**IIb**), (**III**), (**IIIa**), (**IIIb**), (**IIIc**) und **(IV)** zur Behandlung und/oder Prävention des Zustandes nach Operationen, nach Organ- und Gewebs- und Knochenmarktransplantationen, nach plastisch-chirurgischen Operationen, insbesondere solche mit system ischer Anästhesie, nach therapeutischer Bestrahlung mit unterschiedlichen äußeren physikalischen Quellen (wie α-, ß-, γ- Positronen) sowie der Diagnostik und Therapie mit *in vivo* verabreichten Radionukliden-Medikamenten

wobei **U** -H, -**P₃**-**R₃**, -CO-**Z₃**, -**P₃**-**Z₃** oder 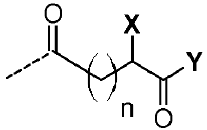 ist

und n, **X, Y, P₃, R₃, R₄,** AS₁, AS₂, AS₃ und **Z₃** die hierin angegebenen Bedeutungen haben.

In einer bevorzugten Ausführungsform betrifft die vorliegende Erfindung Verbindungen der allgemeinen Formeln (**I**), (**II**), (**IIa**), (**IIb**), (**III**), (**IIIa**), (**IIIb**), (**IIIc**) und **(IV)** zur Verwendung in der Behandlung von Ischämie in Kombination mit einem erhöhten CRP-Blutspiegel. Durch eine Ischämie wird der zelluläre Stoffwechsel behindert oder unterbunden. Die durch Drosselung oder Unterbrechung des Blutstroms bewirkte Ischämie geht mit einem Sauerstoffmangel im betroffenen Gebiet einher. Dies kann bis zu einer Nekrose und zu einem Infarkt führen.

In einer bevorzugten Ausführungsform wird die Verbindung der allgemeinen Formeln (**I**), (**II**), (**IIa**), (**IIb**), (**III**), (**IIIa**), (**IIIb**), (**IIIc**) und **(IV)** in der Behandlung und/oder Prävention von Erkrankungen verwendet, die ausgewählt sind aus Herz-Kreislauf-Stillstand, Schlaganfall und Pankreatitis und temporär akut hohe CRP-Blutspiegel aufweisen wobei **U** -H, -**P₃**-**R₃**, -CO-**Z₃**, -**P₃**-**Z₃** oder 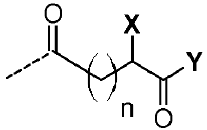 ist und n, **X, Y, P₃, R₃, R₄,** AS₁, AS₂, AS₃ und **Z₃** die hierin angegebenen Bedeutungen haben.

Ein "akut hoher CRP-Blutspiegel" liegt dann vor, wenn die CRP-Konzentration im Blut weit über 20 mg/L liegt. Insbesondere bei akuten Virusinfektionen wie mit SARS-CoV2 sind CRP-Blutspiegel von ungefähr 500 mg/L oder größer über einen Zeitraum von 3 ± 2 Tagen gemessen worden.

In einer weiteren bevorzugten Ausführungsform betrifft die vorliegende Erfindung Verbindungen der allgemeinen Formeln (**I**), (**II**), (**IIa**), (**IIb**), (**III**), (**IIIa**), (**IIIb**), (**IIIc**) und **(IV)** zur Verwendung in der Behandlung und/oder Prävention von Autoimmunerkrankungen, wobei die Autoimmunerkrankung ausgewählt ist aus rheumatoider Arthritis, entzündliche Darmerkrankung, Lupus, Asthma, Diabetes, rheumatische Erkrankungen, AIDS, Abstoßung transplantierter Organe und Gewebe, Rhinitis, chronisch-obstruktive Lungenkrankheiten, Osteoporose, Colitis ulcerosa, Sinusitis, Lupus erythematodes, rekurrierende Infektionen, atopische Dermatitis / Ekzeme und berufsbedingte Allergien, Nahrungsmittelallergien, Arzneimittelallergien, schwere anaphylaktische Reaktionen, Anaphylaxie, Manifestationen allergischer Krankheiten, primäre Immunschwäche, Antikörpermangelzustände, Zell-vermittelte Immunschwäche, schwere kombinierte Immunschwäche, DiGeorge-Syndrom, Hyper-IgE-Syndrom, Wiskott-Aldrich-Syndrom, Ataxia teleangiectasia, Immun-vermittele Krebsformen, Leukozyten-Defekte, Multiple Sklerose (MS), Immun-vermittelter oder Typ 1-Diabetes mellitus, Immun-vermittelte Glomerulonephritis, Sklerodermie, perniziöse Anämie, Alopezie, Pemphigus, Pemphigus vulgaris, Myasthenia gravis, entzündliche Darmerkrankungen, Morbus Crohn, Psoriasis, Autoimmun-Schilddrüsenerkrankungen, Hashimoto-Thyreoditis, Dermatomyositis, Goodpasture-Syndrom, Myasthenia gravis pseudoparalytica, Ophthalmia sympathica, phakogene Uveitis, chronisch-aggressive Hepatitis, primäre biliäre Zirrhose, autoimmunhämolytische Anämie und Morbus Werlhof wobei **U** -H, -**P₃**-**R₃**, -CO-**Z₃**, -**P₃**-**Z₃** oder 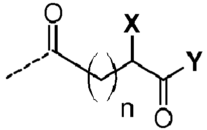 ist und n, **X, Y, P₃, R₃, R₄,** AS₁, AS₂, AS₃ und **Z₃** die hierin angegebenen Bedeutungen haben.

In einer weiteren bevorzugten Ausführungsform betrifft die vorliegende Erfindung Verbindungen der allgemeinen Formeln (**I**), (**II**), (**IIa**), (**IIb**), (**III**), **(IIIa),** (**IIIb**), (**IIIc**) und **(IV)** zur Verwendung in der Behandlung und/oder Prävention von Erkrankungen von Infektionserkrankungen, wobei die Infektionserkrankungen durch Bakterien, Viren, Prionen, Parasiten, Pilze verursacht, induziert, initiiert und/oder verstärkt und/oder durch irritative, traumatische, metabolische, allergische, autoimmunologische oder idiopathische Ursachen ausgelöst werden

wobei **U** -H, -**P₃**-**R₃**, -CO-**Z₃**, -**P₃**-**Z₃** oder 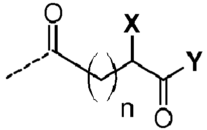 ist und n, **X, Y, P₃, R₃, R₄,** AS₁, AS₂, AS₃ und **Z₃** die hierin angegebenen Bedeutungen haben.

In einer weiteren bevorzugten Ausführungsform betrifft die vorliegende Erfindung Verbindungen der allgemeinen Formeln (**I**), (**II**), (**IIa**), (**IIb**), (**III**), (**IIIa**), (**IIIb**), (**IIIc**) und (**IV**) zur Verwendung in der Behandlung und/oder Prävention von Infektionserkrankungen, wobei die Infektionserkrankung eine durch Coronaviren, insbesondere SARS-CoV-2 ausgelöste Erkrankung ist und ein temporär akut hoher oder chronisch erhöhter CRP-Blutspiegel diagnostiziert wird wobei **U** -H, -**P₃**-**R₃**, -CO-**Z₃**, -**P₃**-**Z₃** oder 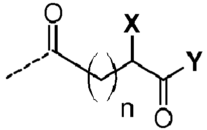 ist

und n, **X, Y, P₃, R₃, R₄,** AS₁, AS₂, AS₃ und **Z₃** die hierin angegebenen Bedeutungen haben.

In einer weiteren Ausführungsform können die hierin beschriebenen Verbindungen der allgemeinen Formeln (**I**), (**II**), (**IIa**), (**IIb**), (**III**), (**IIIa**), (**IIIb**), (**IIIc**) und **(IV)** in Kombination mit extrakorporalen Verfahren zur Senkung des CRP-Spiegels, wie Dialyse oder Apherese verwendet werden. Bei diesen Verfahren wird das CRP aus Blutplasma affinitätschromatographisch mit CRP-bindendem Material beladenen Säulen entfernt. Die Kombination ist nützlich zur Behandlung besonders hoher CRP-Spiegel von über 550 mg/L. Somit betrifft die vorliegende Erfindung Verbindungen der allgemeinen Formeln (**I**), (**II**), (**IIa**), (**IIb**), (**III**), (**IIIa**), (**IIIb**), (**IIIc**) und **(IV)** zur Verwendung als Medikament zur Bindung und/oder Neutralisation von C-reaktivem Protein im Blut in Kombination mit einem Apherese- oder Dialyseverfahren zur Senkung des C-reaktives Protein-Spiegels wobei **U** -H, -**P₃**-**R₃**, -CO-**Z₃**, -**P₃**-**Z₃** oder 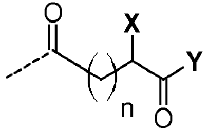 ist

und n, **X, Y, P₃, R₃, R₄,** AS₁, AS₂, AS₃ und **Z₃** die hierin angegebenen Bedeutungen haben.

Ein weiterer Aspekt der vorliegenden Erfindung betrifft pharmazeutische Zusammensetzungen umfassend mindestens eine Verbindung der allgemeinen Formeln (**I**), (**II**), (**IIa**), (**IIb**), (**III**), (**IIIa**), (**IIIb**), (**IIIc**) und (**IV**)

wobei **U** -**P₃**-**R₃**, -CO-**Z₃**, -**P₃**-**Z₃** oder

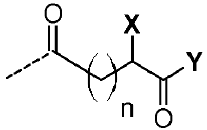

ist

und n, **X, Y, P₃, R₃, R₄,** AS₁, AS₂, AS₃ und **Z₃** die hierin angegebenen Bedeutungen haben.

Zur Verwendung als Medikament zur Bindung und/oder Neutralisation von C-reaktivem Protein im Blut kann die pharmazeutische Formulierung weiter pharmazeutisch verträgliche Träger, Hilfsstoffe und/oder Verdünnungsmittel umfassen. Die hier beschriebenen pharmazeutischen Zusammensetzungen können in einem herkömmlichen festen oder flüssigen Träger oder Verdünnungsmittel und einem herkömmlichen pharmazeutisch hergestellten Hilfsstoff in geeigneter Dosierung in bekannter Weise zubereitet werden.

Die hier beschriebenen pharmazeutischen Zusammensetzungen werden typischerweise zusammen mit geeigneten verträglichen Trägern verabreicht, die im Hinblick auf die beabsichtigte Verabreichungsform ausgewählt werden, z.B. als Pulver zur Konstitution, Gelen, Elixieren, dispergierbaren Granulaten, Sirupen, Suspensionen und dergleichen, und in Übereinstimmung mit konventionellen pharmazeutischen Praktiken.

Ebenfalls inbegriffen sind Zubereitungen in fester Form, die dazu bestimmt sind, kurz vor der Verwendung in eine flüssige Form überführt zu werden. Zu diesen flüssigen Formen gehören Lösungen, Suspensionen und Emulsionen.

Die hier beschriebenen pharmazeutischen Zusammensetzungen können subkutan, durch Spray, durch Injektion, intramuskulär, als Suppositorium oder trans(epi)dermal verabreicht.

Die hierin beschriebenen Verbindungen der allgemeinen Formeln (**I**), (**II**), (**IIa**), (**IIb**), (**III**), (**IIIa**), (**IIIb**), (**IIIc**) und (**IV**) sowie die hierin beschriebenen pharmazeutischen Zusammensetzungen davon sind vorzugsweise für die Behandlung des Menschen gedacht, können aber auch bei Tieren eingesetzt werden und insbesondere bei Pferden und vorzugsweise Reit-, Renn- und Dressurpferden.

In einer bevorzugten Ausführungsform wird die in der hierin beschriebenen pharmazeutischen Zusammensetzung enthaltene Verbindung der allgemeinen Formeln (**I**), (**II**), (**IIa**), (**IIb**), (**III**), (**IIIa**), (**IIIb**), (**IIIc**) und (**IV**) in einem Bereich von 1 mg/kg bis 100 mg/kg, vorzugsweise 2 mg/kg bis 100 mg/kg, vorzugsweise 5 mg/kg bis 100 mg/kg, vorzugsweise 10 mg/kg bis 100 mg/kg pro Körpergewicht pro Tag verabreicht.

In einer weiteren bevorzugten Ausführungsform können die Verbindungen der allgemeinen Formeln (**I**), (**II**), (**IIa**), (**IIb**), (**III**), (**IIIa**), (**IIIb**), (**IIIc**) und (**IV**) sowie eine pharmazeutische Zusammensetzung davon in Kombination mit mindestens einen Komplementblocker zur Bindung und/oder Neutralisation von C-reaktivem Protein im Blut verwendet werden. Vorzugsweise inhibiert der Komplementblocker das/die Komplementprotein(e) C3 und/oder C5, indem er an eine aktive Stelle des/der Komplementproteins C3 und/oder C5 bindet. Der Komplementblocker verhindert zusätzlich die Komplementaktivierung, wodurch Ausbreitung von Schäden und Narben im ischämischen Gewebe gestoppt und eine Infarktnarbe nach Herzinfarkt zusätzlich verringert oder gänzlich verhindert werden.

Somit betrifft die vorliegende Erfindung ebenfalls eine Verbindung der allgemeinen Formeln (**I**), (**II**), (**IIa**), (**IIb**), (**III**), (**IIIa**), (**IIIb**), (**IIIc**) und (**IV**) oder eine pharmazeutische Formulierung davon zur Verwendung als Medikament zur Bindung und/oder Neutralisation von C-reaktivem Protein im Blut, in Kombination mit mindestens einen Komplementblocker

wobei **U** -H, -**P₃**-**R₃**, -CO-**Z₃**, -**P₃**-**Z₃** oder 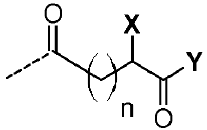 ist

und n, **X, Y, P₃, R₃, R₄** und **Z₃** die hierin angegebenen Bedeutungen haben.

In einer weiteren bevorzugten Ausführungsform können die Verbindungen der allgemeinen Formeln (**I**), (**II**), (**IIa**), (**IIb**), (**III**), (**IIIa**), (**IIIb**), (**IIIc**) und (**IV**) sowie eine pharmazeutische Zusammensetzung davon in Kombination mit mindestens einem CRP-bindenden Konjugat von APPC und humanem Serumalbumin (HSA) verwendet werden, das ein an HSA kovalent gebundenes 4-Aminophenylphosphocholin umfasst

wobei **U** -H, -**P₃**-**R₃**, -CO-**Z₃**, -**P₃**-**Z₃** oder

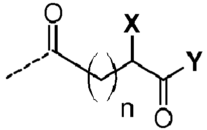

ist

und n, **X, Y, P₃, R₃, R₄** und **Z₃** die hierin angegebenen Bedeutungen haben.

Die hierin beschriebenen CRP-bindende APPC-HSA-Konjugate binden effizient CRP in physiologischen Flüssigkeiten und sind damit besonders zur Behandlung von Erkrankungen geeignet, die mit einem erhöhten CRP-Spiegel assoziiert und/oder durch ihn verursacht werden. Insbesondere sind die hierin beschriebenen APPC-HSA-Konjugate zur Behandlung und/oder Prävention von Erkrankungen geeignet, die durch ein C-reaktives Protein-Blutspiegel von >>5 mg/L verursacht und/oder damit assoziiert sind.

Ein besonderer Vorteil der höhermolekularen APPC-HSA-Konjugate liegt zudem in dessen längerer Verweildauer insbesondere im Blutkreislauf nach intravenöser Injektion, wodurch das besagte Konjugat besonders zur akuten Behandlung von Erkrankungen, wie Infarkten oder Schlaganfällen geeignet ist, bei denen größere Mengen an CRP zunächst in der Blutbahn auftreten.

In einer weiteren bevorzugten Ausführungsform können die Verbindungen der allgemeinen Formeln (**I**), (**II**), (**IIa**), (**IIb**), (**III**), (**IIIa**), (**IIIb**), (**IIIc**) und **(IV)** sowie eine pharmazeutische Zusammensetzung davon in Kombination mit mindestens einem CRP-bindenden APPC-HSA-Konjugat der allgemeinen Formel (V) zur Bindung und/oder Neutralisation von C-reaktivem Protein im Blut verwendet werden, 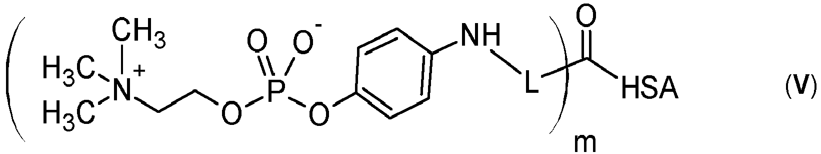 wobei L eine Bindung oder einen Linker darstellt; m eine Zahl zwischen 1 und 100 umfasst und HSA für Humanserumalbumin steht.

Vorzugsweise ist L ein Linker ausgewählt aus: 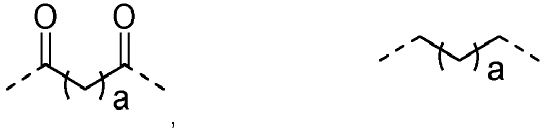 und 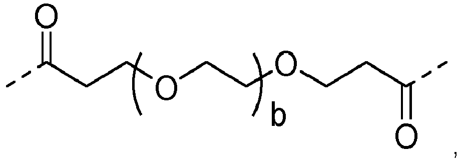 wobei a eine ganze Zahl ausgewählt aus 1, 2, 3, 4, 5, 6, 7, 8, 9 und 10 ist, b eine ganze Zahl ausgewählt aus 1, 2, 3 und 4 ist.

Vorzugsweise beträgt **m** zwischen 1 und 100, noch bevorzugter zwischen 1 und 50, noch bevorzugter zwischen 1 und 30, noch bevorzugter zwischen 1 und 20, noch bevorzugter zwischen 2 und 15 und am bevorzugtesten zwischen 5 und 10. Ebenfalls bevorzugt ist, wenn **m** zwischen 35 und 40, noch bevorzugter zwischen 30 und 35, noch bevorzugter zwischen 25 und 30, noch bevorzugter zwischen 20 und 25, noch bevorzugter zwischen 15 und 20, noch bevorzugter zwischen 10 und 15, noch bevorzugter zwischen 5 und 10 und am bevorzugtesten zwischen 1 und 5 liegt. Ebenso ist bevorzugt, wenn **m** ungefähr 1, 2, 3, 4, 5, 6, 7, 8, 9, 10, 11, 12, 13, 14, 15, 16, 17, 18, 19 oder 20 beträgt. Weiterhin ist bevorzugt, wenn **m** nicht größer als 3, noch bevorzugter nicht größer als 4, noch bevorzugter nicht größer als 5, noch bevorzugter nicht größer als 6, noch bevorzugter nicht größer als 7, noch bevorzugter nicht größer als 8, noch bevorzugter nicht größer als 9, noch bevorzugter nicht größer als 10 ist.

Die hierin beschriebenen APPC-HSA-Konjugate sind in der Lage, so an das CRP zu binden, dass eine anschließende Komplexierung mit dem Komplementprotein C1q nicht mehr oder nur in geringerem stattfindet. Dadurch wird die proödematöse und pronekrotische Wirkung von CRP bei Erkrankungen wie Herzinfarkt und Schlaganfall gestoppt, verringert oder gar ausgesetzt. Letztlich kann so die Ausbreitung von Schäden und Narben im ischämischen Gewebe gestoppt und eine Infarktnarbe nach Herzinfarkt verringert oder gänzlich verhindert werden.

Um die Aggregation von CRP-gebundenen Phosphocholin-Konjugaten zu unterbinden und damit das Risiko einer Glomerulonephritis zu minimieren, ist das molekulare Verhältnis **m** von 4-Aminophenylphosphocholin zu Humanserumalbumin im APPC-HSA-Konjugat in den Konjugaten der allgemeinen Formel (**IV**) vorzugsweise nicht größer als 3, noch bevorzugter nicht größer als 4, noch bevorzugter nicht größer als 5, noch bevorzugter nicht größer als 6, noch bevorzugter nicht größer als 7, noch bevorzugter nicht größer als 8, noch bevorzugter nicht größer als 9, noch bevorzugter nicht größer als 10.

Somit betrifft die vorliegende Erfindung ebenfalls eine Verbindung der allgemeinen Formeln (**I**), (**II**), (**IIa**), (**IIb**), (**III**), (**IIIa**), (**IIIb**), (**IIIc**) und (**IV**) oder eine pharmazeutische Formulierung davon zur Verwendung als Medikament zur Bindung und/oder Neutralisation von C-reaktivem Protein im Blut, in Kombination mit mindestens einem CRP-bindenden APPC-HSA-Konjugat der allgemeinen Formel (V) wobei **U** -H, -**P₃**-**R₃**, -CO-**Z₃**, -**P₃**-**Z₃** oder 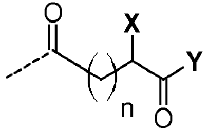 ist und n, **m, L, X, Y, P₃, R₃, R₄,** AS₁, AS₂, AS₃ und **Z₃** die hierin angegebenen Bedeutungen haben.

In einer weiteren bevorzugten Ausführungsform können die Verbindungen der allgemeinen Formeln (**I**), (**II**), (**IIa**), (**IIb**), (**III**), (**IIIa**), (**IIIb**), (**IIIc**) und (**IV**) sowie eine pharmazeutische Zusammensetzung davon in Kombination mit mindestens einem CRP-bindenden APPC-HSA-Konjugat der allgemeinen Formel (**VI**) zur Bindung und/oder Neutralisation von C-reaktivem Protein im Blut verwendet werden 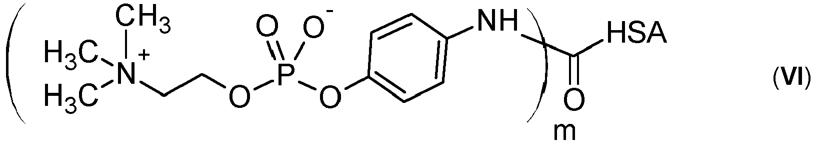 wobei **m** eine Zahl zwischen 1 und 100 umfasst und HSA für Humanserumalbumin steht.

Vorzugsweise beträgt **m** zwischen 1 und 100, noch bevorzugter zwischen 1 und 50, noch bevorzugter zwischen 1 und 30, noch bevorzugter zwischen 1 und 20, noch bevorzugter zwischen 2 und 15 und am bevorzugtesten zwischen 5 und 10. Ebenfalls bevorzugt ist, wenn **m** zwischen 35 und 40, noch bevorzugter zwischen 30 und 35, noch bevorzugter zwischen 25 und 30, noch bevorzugter zwischen 20 und 25, noch bevorzugter zwischen 15 und 20, noch bevorzugter zwischen 10 und 15, noch bevorzugter zwischen 5 und 10 und am bevorzugtesten zwischen 1 und 5 liegt. Ebenso ist bevorzugt, wenn **m** ungefähr 1, 2, 3, 4, 5, 6, 7, 8, 9, 10, 11, 12, 13, 14, 15, 16, 17, 18, 19 oder 20 beträgt. Weiterhin ist bevorzugt, wenn **m** nicht größer als 3, noch bevorzugter nicht größer als 4, noch bevorzugter nicht größer als 5, noch bevorzugter nicht größer als 6, noch bevorzugter nicht größer als 7, noch bevorzugter nicht größer als 8, noch bevorzugter nicht größer als 9, noch bevorzugter nicht größer als 10 ist.

Somit betrifft die vorliegende Erfindung ebenfalls eine Verbindung der allgemeinen Formeln (**I**), (**II**), (**IIa**), (**IIb**), (**III**), (**IIIa**), (**IIIb**), (**IIIc**) und (**IV**) oder eine pharmazeutische Formulierung davon zur Verwendung als Medikament zur Bindung und/oder Neutralisation von C-reaktivem Protein im Blut, in Kombination mit mindestens einem CRP-bindenden APPC-HSA-Konjugat der allgemeinen Formel (**VI**) wobei **U** -H, -**P₃**-**R₃**, -CO-**Z₃**, -**P₃**-**Z₃** oder 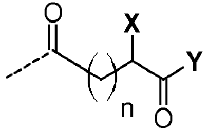 ist und n, **m, X, Y, P₃, R₃, R₄,** AS₁, AS₂, AS₃ und **Z₃** die hierin angegebenen Bedeutungen haben.

Die hier beschriebenen APPC-HSA-Konjugate der Formeln (**V**) und (**VI**) können aus Phosphocholin und Humanserumalbumin mit den aus dem Stand der Technik bekannten Methoden zur Synthese von Peptidkonjugaten hergestellt werden. Humanserumalbumin weist eine dem Fachmann bekannte Vielzahl an funktionellen Gruppen auf, über die das Phosphocholin kovalent gebunden werden kann. Diese funktionelle Gruppe kann u.a. eine primäre Aminogruppe eines Lysinrestes sein, die mit einem Aktivester reagiert, eine Carboxylgruppe eines Glutamat- oder Aspartat-Restes oder eine Thiolgruppe eines Cysteinrestes.

Insbesondere Peptidkupplungsreagenzien wie Carbodiimide (einschließlich Dicyclohexylcarbodiimid (DCC), Diisopropylcarbodiimid (DIC) und 1-Ethyl-3-(3-dimethylaminopropyl)carbodiimid (EDC)), Phosphoniumsalze (einschließlich BOP-Reagenz, PyBOP, PyBrOP und PyOxim), Immoniumsalze (einschließlich BOMI und BDMP), Aminiumsalze (einschließlich HBTU, TBTU, HATU, HCTU und TATU), Uroniumsalze (einschließlich TNTU, TPTU, TOTU, TDBTU, COMU, COMBU, TOMBU und TSTU), Imidazoliumsalze (einschließlich CBMIT, BOI, CIP, CIB und CMBI) oder Carbonyldiimidazol sind für die kovalente Bindung des APPC an eine funktionelle Gruppe der Aminosäureseitenkette des HSA geeignet.

Ebenfalls für die Kupplung von APPC an HSA sind Aktivester geeignet. Bevorzugte Aktivester sind N-(γ-Maleimidobutyryloxy) sulfosuccinimidester (sulfo-GMBS), Succinimidyl (4-iodacetyl) aminobenzoat (sulfo-SIAB), Succinimidyl-3-(bromacetamido)propionat (SBAP), Disuccinimidylglutarat (DSG), Disuccinimidyladipat (DSA), 2-Pyridyldithiol-tetraoxatetradecan-N-hydroxysuccinimid (PEG-4-SPDP), Bis-(4-nitrophenyl)adipat und Bis-(4-nitrophenyl)succinat.

In Abwesenheit eines Linkers kann das 4-Aminophenylphosphocholin direkt an eine Carboxylgruppe eines Glutamat- oder Aspartat-Restes mit einem Peptidkupplungsreagenz kovalent gebunden werden. Für die Einbringung eines Linkers kann zunächst eine funktionelle Gruppe des HSA (Carboxylat) oder das aromatische Amin des APPC mit einem Peptidkupplungsreagenz oder Aktivester aktiviert und anschließend mit dem Linker zur Reaktion gebracht werden.

### Figurenbeschreibung

- **Figur 1**: ELISA zur Bindung von huCRP an Festphasen-gebundenes Biotin-d-Glu(APPC)-NH₂ (**2)** mit steigender CRP-Konzentration. Mittels eines MAK gegen humanes CRP wurde die Menge an gebundenem huCRP detektiert.
- **Figur 2**: ELISA zur Bindung von freiem CRP an freies Biotin-d-Glu(APPC)-NH₂ **(2).** In Lösung hat zuvor **(2)** an freies huCRP gebunden. Über die Fixierung von (**2)** auf Streptavidin beschichteten MTP erfolgt der Nachweis über die Detektion des an (**2)** gebundenen huCRP.

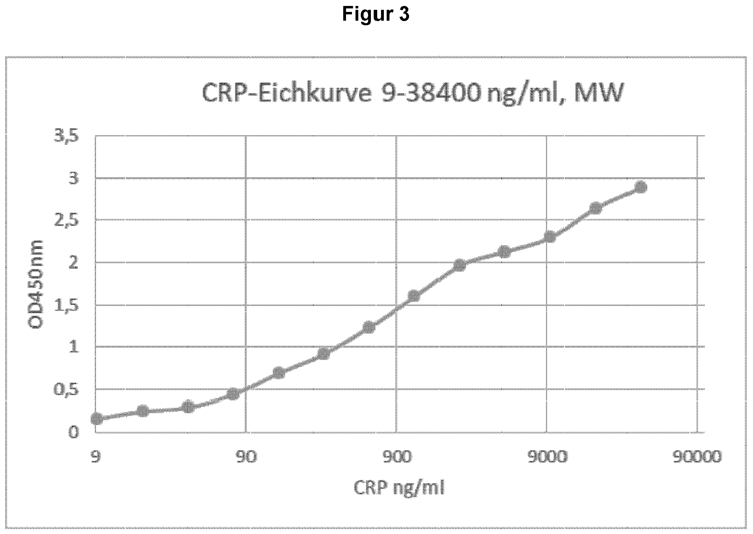

- **Figur** 3: ELISA zur Bindung von huCRP an Festphasen-gebundenes Biotin-PEG-I-Glu(APPC)-NH₂ (**3)** mit steigender CRP-Konzentration. Mittels eines MAK gegen humanes CRP wurde die Menge an gebundenem CRP detektiert.

### Beispiele

### Abkürzungen

- APPC:: 4-Aminophenylphosphocholin, Ligand für CRP
- Äq.:: Äquivalent
- BSA:: Bovines Serumalbumin
- CRP:: C Reaktives Protein
- ELISA:: Enzyme-Linked Immuno Sorbent Assay
- HSA:: Humanserumalbumin
- huCRP:: humanes CRP
- IgG:: Immunglobulin G
- Kav.:: Kavität
- MAK.: Monoklonaler Antikörper
- MTP:: Mikrotiterplatte
- Opp:: 2-phenylisopropyl
- PBS:: Phosphat gepufferte physiologische Kochsalzlösung
- POD-anti Maus:: Peroxidase, gekoppelt an einen Sekundärantikörper gegen murines IgG
- S:: Laborschüttler für MTP
- TMB:: 3,3',5,5' Tetramethylbenzidin, Substrat für die POD, Farbreagenz
- Tween20:: Detergenz, Handelsname

### Beispiel 1: Synthese von APPC-gebundenem D-Glutaminsäureamid (D-Glu(APPC)-NH₂) = Verbindung 1

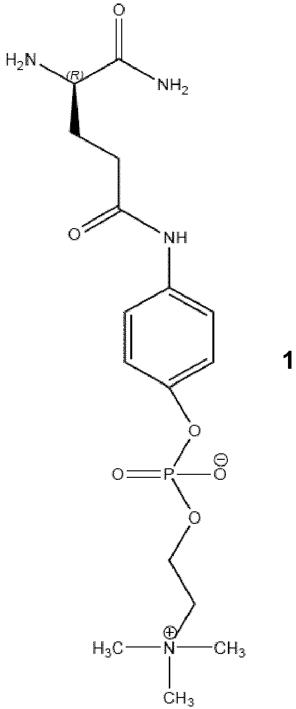

D-Glu(APPC)-NH₂**(1)** wurde wie folgt synthetisiert:
D-Glutaminsäureamid wurde in Lösung in Gegenwart von 1 Äq. 1-Ethyl-3-(3-dimethylaminopropyl)carbodiimid aktiviert und anschließend 1 Äq. 4-Aminophenylphosphorylcholin hinzugegeben. Das Reaktionsgemisch wurde über Nacht gerührt und anschließend aufgearbeitet. Verbindung 1 wurde als farbloser Feststoff nahezu quantitativ erhalten.

### Beispiel 2: Synthese von biotinyliertem APPC-gebundenem D-Glutaminsäure-amid (Biotin-D-Glu(APPC)-NH₂) = Verbindung 2

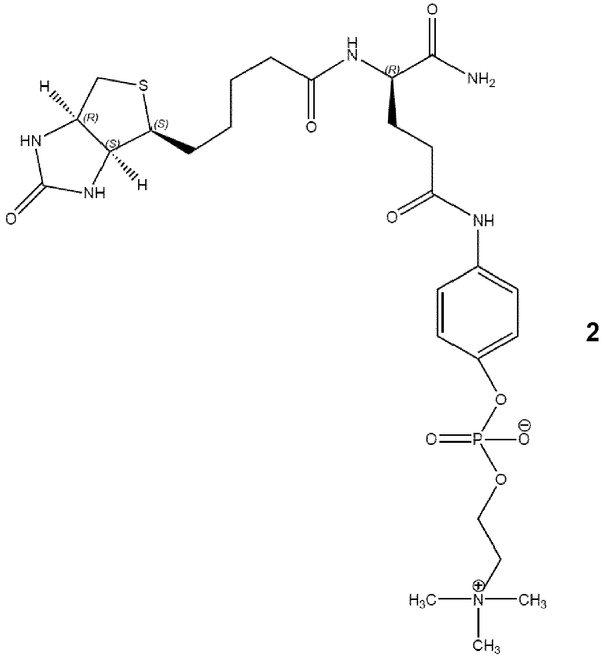

Biotin-D-Glu(APPC)-NH₂ (2) wurde analog zu (1) wie folgt synthetisiert:
Biotin-D-Glutaminsäureamid wurde in Lösung in Gegenwart von 1 Äq. 1-Ethyl-3-(3-dimethylaminopropyl)carbodiimid aktiviert und anschließend 1 Äq. 4-Aminophenylphosphorylcholin hinzugegeben. Das Reaktionsgemisch wurde über Nacht gerührt und anschließend aufgearbeitet. Verbindung 2 wurde als farbloser Feststoff nahezu quantitativ erhalten.

### Beispiel 3: Assay zur Bindung von CRP an Festphasen-fixiertes Biotin-D-Glu(APPC)-NH₂ (Verbindung 2)

Mit Streptavidin beschichtete Mikrotiterplatten wurden mit einer Lösung enthaltend Biotin-D-Glu(APPC)-NH₂) **(2)** inkubiert und anschließend mit Phosphatpuffer (PBS) gewaschen Anschließend wurden die reaktiven Teile der MTP-Oberfläche mit BSA-Lösung geblockt (ein Standardverfahren). Danach wurde CRP im Konzentrationsbereich von 31-500 ng/ml dazugegeben und inkubiert (s. Tabelle unten). Nach erneuter Waschung erfolgte die Detektion des gebundenen CRP mittels eines murinen monoklonalen Antikörpers (DH7) gegen CRP und eines sekundären, POD-konjugierten Antimaus-Antikörpers sowie anschließender Substratentwicklung.

**Tabelle 1: Daten der zugrunde liegenden CRP-Bindungskurve (Mittelwerte aus Doppelbestimmungen)**

| **CRP [ng/ml]** | **Optische Dichte (OD)** |
|---|---|
| 500 | 3,242 |
| 250 | 2,627 |
| 125 | 2,081 |
| 62,5 | 1,512 |
| 31 | 1,061 |
| 0 | 0,173 |

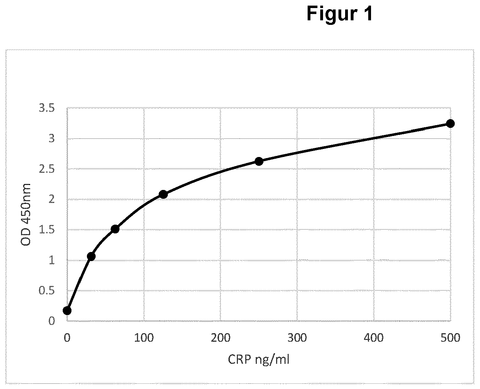

Die CRP-Bindungskurve in Figur 1 zeigt, dass Verbindung 2 an CRP bindet. Somit erfüllt die Verbindung die Anforderungen eines niedermolekularen pharmakologischen CRP-Blockers.

### Beispiel 4: Assay zur Bindung von freiem CRP und freiem Biotin-d-Glu(APPC)-NH₂ (2)

CRP und Verbindung **2** wurden zur Komplexierung in einem Reaktionsgefäß 1 h bei Raumtemperatur inkubiert. Die finalen Konzentrationen betrugen dabei 10 µg/ml CRP (MGW ca. 125 KD) und 2 µg/ml Verbindung **2** (MGW ca. 1 KD). Anschließend wurde die Lösung in den in der **Tab. 2** angegebenen Verdünnungsstufen zu je 100 µl/ml in mit Streptavidin beschichtete Mikrotiterplatten (MTP) gegeben und mit einer Blocklösung (Roche) 30 min bei Raumtemperatur inkubiert. Die MTP wurden danach 3 mal gewaschen. Mittels des für huCRP spezifischen *in house* monoklonalen Antikörpers "DH7" und einem Peroxidase konjugierten Sekundärantikörper (AntiMaus) sowie dem Substrat TMB wurde das zuvor an Verbindung **2** gebundene huCRP detektiert.

**Tabelle 2: Verdünnungsreihe CRP : 2 und deren Optische Dichte (Mittelwerte aus Doppelbestimmungen)**

| **Komplexverdünnung CRP : Verbindung 2** | **Optische Dichte (OD)** |
|---|---|
| 1:20 | 2,685 |
| 1:40 | 2,219 |
| 1:80 | 1,801 |
| 1:160 | 1,242 |
| 1:320 | 0,875 |
| 1:640 | 0,548 |
| 1:1280 | 0,381 |
| 0 | 0,144 |

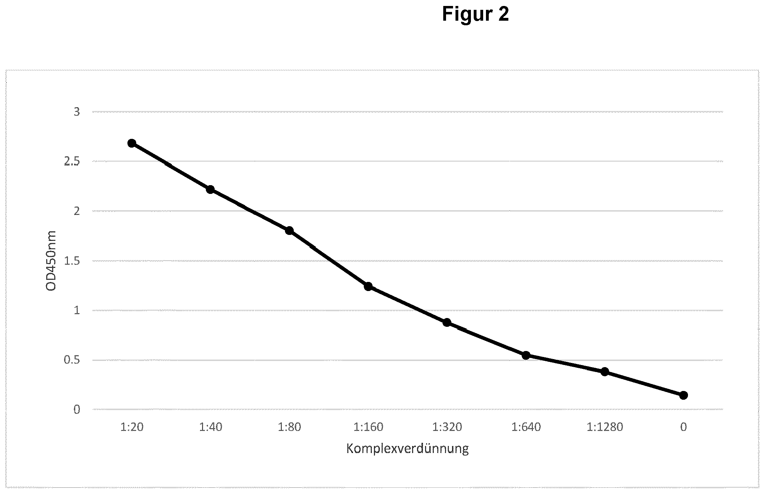

Verbindung **2** bindet CRP in Lösung und als Komplex an das Streptavidin der Mikrotiterplatte. Das ergibt sich auch aus der nahezu linearen Verdünnungsreihe in Figur 2.

### Beispiel 5: Synthese von PEGylierter L-Glutaminsäure (Biotin-PEG-L-Glu(APPC)-NH₂) Verbindung 3

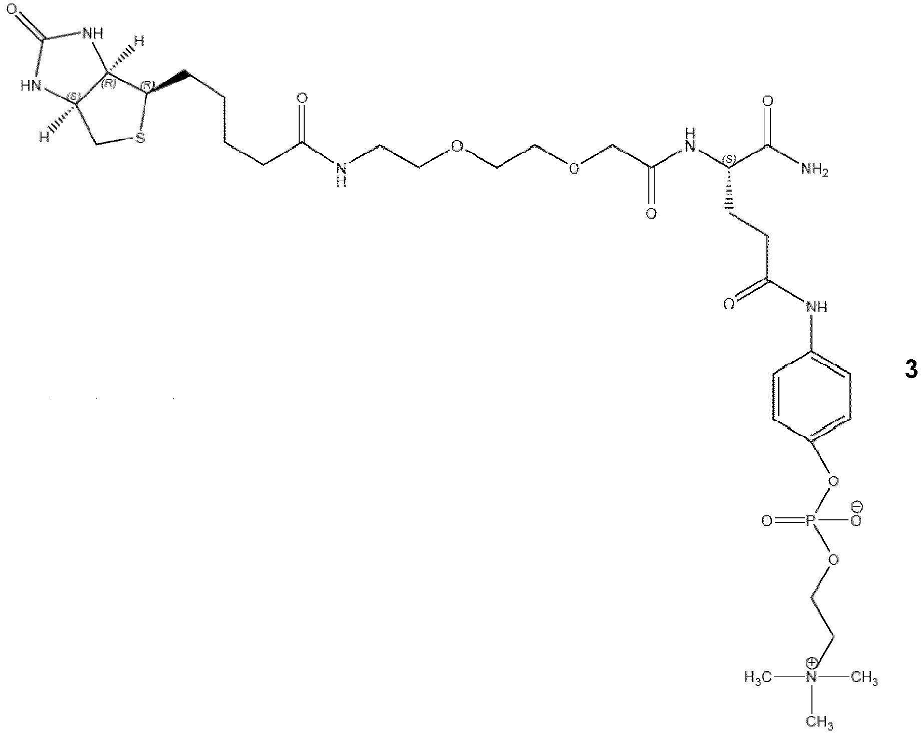

Verbindung **3** wurde unter Standardbedingungen der Fmoc-Festphasensynthese wie folgt synthetisiert:
Fmoc-L-Glu(Opp)-OH wurde an fester Phase (TentaGel^{®} HL RAM, 0,37 mMol/g) gebunden. Die Fmoc-Gruppe wurde durch Zugabe von Piperidin abgespalten und anschließend mit Biotin-PEG₂-NHS (9-(Biotinamido)-4,7-dioxanonansäure-N-succinimidester) versetzt. Die 2-phenylisopropyl-Gruppe (Opp) wurde durch Zugabe von Säure abgespalten und anschließend mit 4-Aminophenylphosphorylcholin und
HATU (O-(7-Azabenzotriazol-1-yl)-N,N,N',N'-tetramethyluronium-hexafluorphosphat]) in Gegenwart einer Base versetzt. Die Abspaltung vom Harz erfolgte dann mittels TFA, Wasser und Triethylsilan. Nach Aufreinigung mittels präparativer HPLC (C18) wurde die Verbindung 3 mit einer Reinheit > 95% erhalten.

### Beispiel 6: Assay zur Bindung von freiem CRP an Festphasen-fixiertes (Biotin-PEG-L-Glu(APPC)-NH₂) (Verbindung 3)

Der Assay wurde in 7 Schritten durchgeführt. Ein schematischer Ablauf ist in **Tabelle 4** abgebildet:

**Tabelle 3 Optische Dichte (OD bei 450 nm) der Bindung von steigenden CRP-Konzentrationen an fixiertes 3; MW = Mittelwert.**

| CRP ng/ml | OD | OD | MW |
|---|---|---|---|
| 38400 | 2,824 | 2,944 | 2,884 |
| 19200 | 2,653 | 2,626 | 2,6395 |
| 9600 | 2,273 | 2,316 | 2,2945 |
| 4800 | 2,057 | 2,193 | 2,125 |
| 2400 | 1,936 | 1,989 | 1,9625 |
| 1200 | 1,563 | 1,632 | 1,5975 |
| 600 | 1,221 | 1,249 | 1,235 |
| 300 | 0,881 | 0,96 | 0,9205 |
| 150 | 0,669 | 0,716 | 0,6925 |
| 75 | 0,428 | 0,458 | 0,443 |
| 37,5 | 0,28 | 0,299 | 0,2895 |
| 18,7 | 0,214 | 0,27 | 0,242 |
| 9,3 | 0,147 | 0,16 | 0,1535 |

**Tab.4. Schematischer Ablauf der Versuchsdurchführung**

| **Nr** | **Reagenz** | **Konz.** | **µl/Kav.** | **Puffer** | **Inkubation** | **Waschen** |
|---|---|---|---|---|---|---|
| **1** | Verbindg. 3 (2µg/ml) in jedes well von Streptavidin-beschichteten Mikrotiterplatten (Standard Capacity) von Fa. BIOTEZ Berlin pipettieren | Final 2 µg/ml, gelöst mit 8 µl DMSO, danach + 1 ml PBS aliquotiert und wieder lyophilisiert | 100 | 0,05 M PBS + 0,01% BSA + 0,045% ProClin 300*, pH 7,2) 0,05 M PBS = 250 ml Wasser +0,47 g KH₂PO₄+1,62 g Na₂HPO₄*2H₂O+ 2,2 g NaCl | 1h RT + S | 2x PBS/Tween |
| **2** | CRP (1,27mg/ml) | 38.400-0 ng/ml | 100 | 25 mM Tris/HCI (pH8,0), 0,9 % NaCl,0,1 % Tween 20,0,1 % BSA,5 mM CaCl₂ | 1h RT + S | 3x |
| **3** | Monoklonaler Antikörper (DH7) gegen CRP (gereinigt 500µg/ml) (Verdünnung 1:100) | Final 5µg/ml | 100 | 25 mM Tris/HCI (pH7,3), 0,9 % NaCl,0,1 % Tween 20, 0,1 % BSA | 1h RT + S | 3x |
| **4** | POD-anti Maus IgG (Sigma) | 1:3000 | 100µl | POD anti Maus IgG mit CRP-Puffer pH 7,3 | 1h RT + S | 3x |
| **5** | Substrat: TMB Seramun | Ready for use | 100 | 100µl SeramunBlaufast | 13 ±2 min, RT, im Dunklen | |
| **6** | Stopplösung: H₂SO₄ | 0,25 M | 50 | 0,25 M H₂SO₄ | | |
| **7** | Messung der Optischen Dichte (OD) der Farblösung: 450 nm (Ref.: 620 nm) | | | | | |

| | | | | | | |
|---|---|---|---|---|---|---|
| * ProClin 300: 1:1 Gemisch aus 5-Chlor-2-methyl-1,2-thiazol-3-on und 2-Methyl-1,2-thiazol-3-on | | | | | | |

1. In Streptavidin beschichtete Mikrotiterplatten wurden pro Kavität je 100 µl einer Lösung von **3** (finale Konzentration 2 µg/ml) gegeben und 1 Stunde (h) bei Raumtemperatur (RT, 20-22 °C) inkubiert. Anschließend wurden die Kavitäten mit je 200 µl PBS/Tween Puffer 2 x gewaschen.

2. Frisch präpariertes humanes CRP wurde, ausgehend von einer Stammlösung (1,27 mg/ml in physiologscher Kochsalzlösung), in die primäre Konzentration halbierenden Verdünnungsschritten (Verdünnungspuffer: 25 mM Tris/HCl, pH 8,0) im Bereich von 38.400 - 9,3 ng/ml in die Kavitäten (je 100 µl/ml) gegeben. Die Mikrotiterplatten wurden 1 h bei RT inkubiert und 3 x mit 200 µl PBS/Tween Puffer gewaschen, um das überschüssige CRP zu entfernen.

3. Die Detektion des gebundenen CRP erfolgte mittels eines gereinigten murinen gegen humanes CRP gerichteten monoklonalen Antikörpers (MAK). Der MAK wurde in einer Endkonzentration von 5 µg/ml (Verdünnungspuffer: 25 mM Tris/HCl) zu je 100 µl zugegeben und 1 h bei RT inkubiert. Anschließend wurden die Kavitäten der MTP 3 x gewaschen, um den überschüssigen, nicht gebunden MAK zu entfernen.

4. Das an **3** gebundene CRP wurde durch Inkubation der Kavitäten mit je 100 µl Peroxidase markierten Antikörper gegen Maus IgG (1:3.000 Verdünnung, Fa. Sigma), im "CRP-Puffer" markiert. Die Inkubationszeit betrug 1 h bei RT. Der überschüssige Antikörper wurde durch 3 maliges Waschen der Kavitäten mit "CRP-Puffer" entfernt.

5. Als Substrat für die Peroxidase (POD) wurden 100 µl 3,3',5,5'-Tetramethylbenzidin (TMB, Fa. Seramun, SeramunBlau^{®}fast) pro Kavität eingesetzt. Dabei wurde das farblose Substratmolekül durch POD in einen photometrisch messbaren blauen Farb-komplex umgewandelt. Die Substratinkubation erfolgte in der Dunkelheit innerhalb von 12±2 min bei RT.

6. Die enzymatische Reaktion wurde durch Zugabe von je 50 µl 0,25 M Schwefelsäure unterbrochen.

7. Unmittelbar danach erfolgte die photometrische Messung der optischen Dichte in einem Mikrotiterplatten-Photometer bei einer Wellenlänge von 450 nm (gegen eine Referenz von 620 nm). Die Farbintensität als optische Dichte (OD) entspricht dabei der Menge des vorgelegten CRP. Ergebnisse sind in **Tabelle 3** dargestellt:

### Beispiel 7: Synthese von doppelt PEGylierter d-Glutaminsäure (PEG-I-Glu(APPC)-PEG-NH₂ ) = Verbindung 4

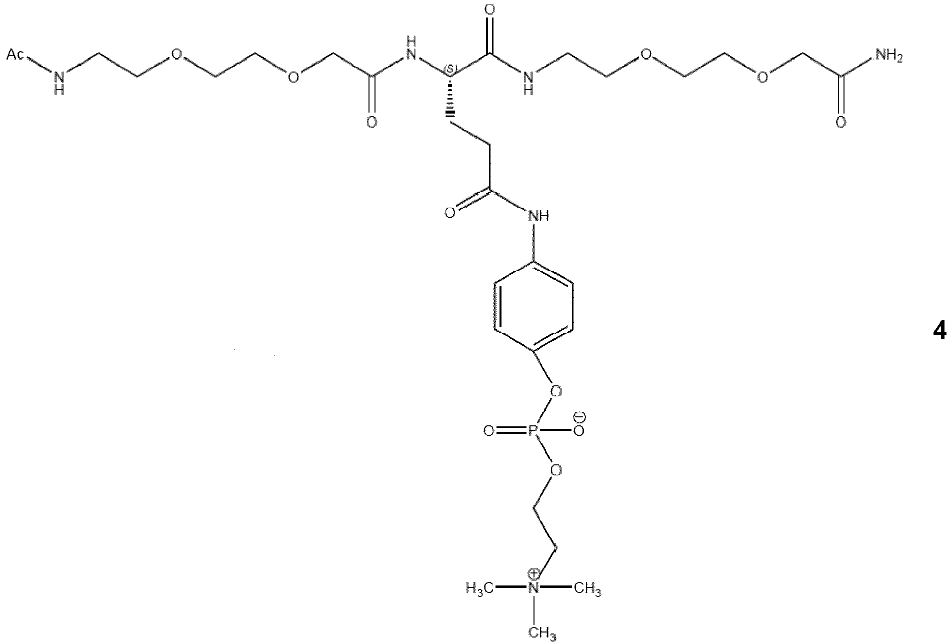

Verbindung **4** wurde analog zu Verbindung **3** nach den Standardbedingungen der Fmoc-Festphasensynthese hergestellt.

## Patentansprüche

1. Eine Verbindung der allgemeinen Formel (**I**): 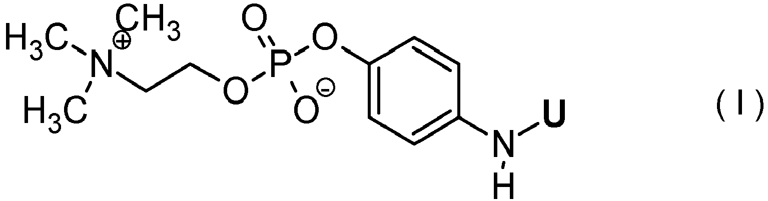 wobei U ausgewählt ist aus ―**P₃**―**R₃**, ―CO―**Z₃**, ―**P₃**―**Z₃** oder 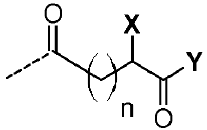 wobei n eine ganze Zahl ausgewählt aus 1, 2 und 3 ist;
**X** ―NH₂, ―NH―**P₁**―**R₁,** ―NH―CO―**Z₁** oder ―NH―**P₁**―**Z₁** darstellt,
**Y** ―OH, ―NH₂, ―NH―**P₂**―**R₂**, ―NH―**Z₂** oder ―NH―**P₂**―**Z₂** darstellt,
**P₁**―H**, P₂**―H und **P₃**―H unabhängig voneinander aus 1 ― 4 Aminosäuren bestehende Peptidreste darstellen,
**Z₁**, **Z₂** und **Z₃** unabhängig voneinander ―(CH₂)ₒ₁―(O―C₂H₄)ₒ₂―O―(CH₂)ₒ₃―**T** darstellen, wobei
o1 eine ganze Zahl ausgewählt aus 1, 2, 3, 4, 5 und 6 ist
o2 eine ganze Zahl ausgewählt aus 1, 2, 3, 4, 5, 6, 7, 8, 9 und 10 ist;
o3 eine ganze Zahl ausgewählt aus 1, 2, 3, 4, 5 und 6 ist;
**T** ―H, ―OCH₃, ―CO―NH₂ oder ―NH₂ darstellt;
**R₁**, **R₂** und **R₃** unabhängig voneinander ausgewählt sind aus ―H, ―CH₃, ―CH₂CH₃, ―CO―CH₃ und ―CO―CH₂CH₃,
sowie Enantiomere, stereoisomere Formen, Mischungen von Enantiomeren, Diastereomere, Mischungen von Diastereomeren, Prodrugs, Hydrate, Solvate, Tautomere, Racemate der vorgenannten Verbindungen und pharmazeutisch verträgliche Salze davon.

2. Die Verbindung gemäß Anspruch 1 mit der Struktur der allgemeinen Formel (**II**) 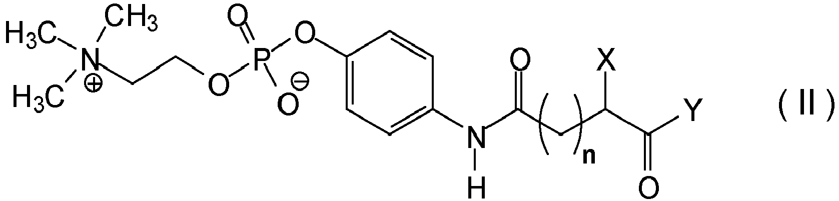 wobei n 2 ist und X und Y die in Anspruch 1 angegebenen Bedeutungen haben.

3. Die Verbindung gemäß Anspruch 2 mit der Struktur der allgemeinen Formel (**II**), wobei n 2 ist,
**Y** ―OH oder ―NH₂ darstellt,
**X** ―NH₂ oder ―NH―CO―**Z₁** darstellt, wobei
**Z₁** ―(CH₂)ₒ₁―(O―C₂H₄)ₒ₂―O―(CH₂)ₒ₃―**T** ist, mit
o1 1 oder 2 ist,
o2 eine ganze Zahl ausgewählt aus 1, 2, 3, 4 und 5 ist;
o3 1 oder 2 ist und
**T** ―NH₂ darstellt.

4. Die Verbindung gemäß Anspruch 2 oder 3 mit der Struktur der allgemeinen Formel (**II**),
wobei n 2 ist,
**X** ―NH₂ oder ―NH―**P₁**―**R₁** darstellt,
**Y** ―OH, ―NH₂ oder ―NH―**P₂**―**R₂** darstellt,
**P₁** und **P₂** unabhängig voneinander ―AS₁―, ―AS₁―AS₂― oder ―AS₁―AS₂―AS₃― darstellen und AS₁, AS₂ und AS₃ für jedes Vorkommen unabhängig voneinander Aminosäuren der folgenden Struktur darstellen: 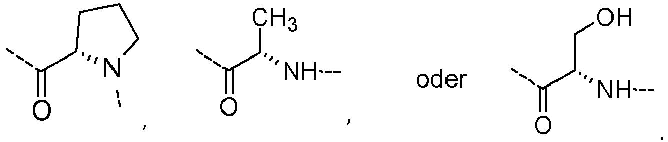 **R₁** und **R_{2'}** die in Anspruch 1 angegebenen Bedeutungen aufweisen.

5. Die Verbindung gemäß einem der Ansprüche 2 bis 4 mit der Struktur der allgemeinen Formel (**II**),
wobei n 2 ist, **Y** ―OH und **X** ―NH₂ sind.

6. Die Verbindung gemäß Anspruch 1 mit der Struktur der allgemeinen Formel (**III**) 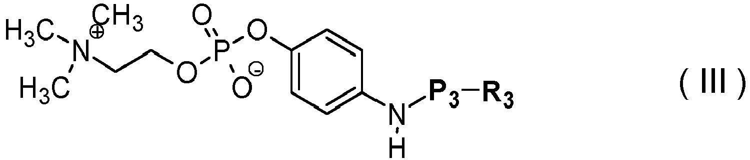 wobei **P₃** ausgewählt ist aus ―**Q₁**―, ―**Q₁**―**Q₂**―, ―**Q₁**―**Q₂**―**Q₃**― und ―**Q₁**―**Q₂**―**Q₃**―**Q₄**―;
und **Q₁**, **Q₂**, **Q₃** und **Q₄** unabhängig voneinander ausgewählt sind aus 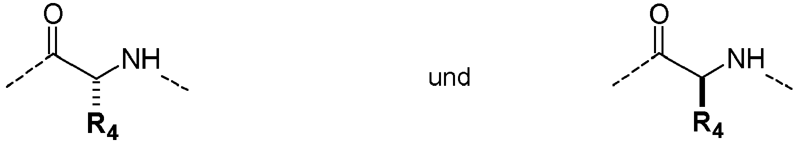 **R₄** unabhängig für jedes Vorkommen ―H, ―CH₃, ―CH₂OH, ―CH₂SH, ―COOH, ―CONH₂, ―CH(OH)CH₃, ―CH(CH₃)₂, ―CH₂―CH(CH₃)₂, ―CH(CH₃)―C₂H₅, ―CH₂―C₆H₅, ―CH₂―C₆H₄―OH, ―C₂H₄―S―CH₃, ―CH₂―COOH, ―CH₂―CONH₂, ―C₂H₄―COOH, ―C₂H₄―CONH₂, ―C₃H₆―NH―C(NH)―NH₂, ―C₄H₈―NH₂, 5-imidazolyl oder 2-indolyl darstellt, oder **R₄** und das benachbarte Stickstoffatom zusammen mit den Atomen, an die sie gebunden sind, einen Pyrrolidin-Ring bilden
und **R₃** die in Anspruch 1 angegebene Bedeutung hat.

7. Die Verbindung gemäß Anspruch 6 mit der Struktur der allgemeinen Formel (**III**), wobei **P₃** 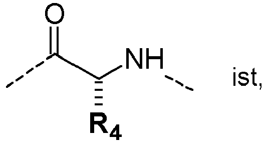
**R₄** unabhängig für jedes Vorkommen ―H, ―CH₂―COOH, ―CH₂―CONH₂, ―C₂H₄―COOH, ―C₂H₄―CONH₂ oder ―C₂H₄―S―CH₃, darstellt, oder
**R₄** und das benachbarte Stickstoffatom zusammen mit den Atomen, an die sie gebunden sind, einen Pyrrolidin-Ring bilden.

8. Eine pharmazeutische Zusammensetzung enthaltend mindestens eine Verbindung gemäß einem der Ansprüche 1 bis 7.

9. Die pharmazeutische Zusammensetzung gemäß Anspruch 8 weiter umfassend einen pharmazeutisch verträglichen Träger, ein Hilfsstoff und/oder ein Verdünnungsmittel.

10. Eine Verbindung der allgemeinen Formel (**I**) oder eine pharmazeutische Zusammensetzung davon zur Verwendung zur Behandlung und/oder Prävention von Erkrankungen, die durch einen CRP-Blutspiegel von >20 mg/L verursacht werden und/oder damit assoziiert sind, 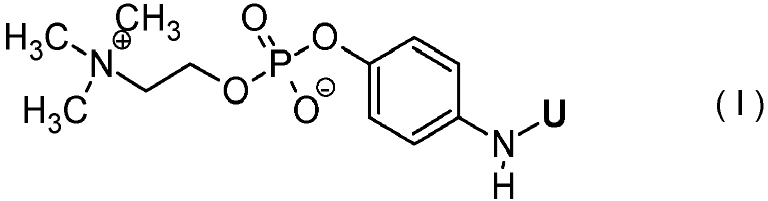 wobei **U** ―H, ―**P₃**―**R₃,** ―CO―**Z₃,** ―**P₃**―**Z₃** oder 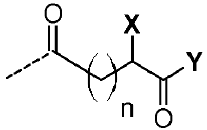 ist n eine ganze Zahl ausgewählt aus 1, 2 und 3 ist;
**X** ―NH₂, ―NH―**P₁**―**R₁**, ―NH―CO―**Z₁** oder ―NH―**P₁**―**Z₁** darstellt,
**Y** ―OH, ―NH₂, ―NH―**P₂**―**R₂**, ―NH―**Z₂** oder ―NH―**P₂**―**Z₂** darstellt,
**P₁**―H**, P₂**―H und **P₃**―H unabhängig voneinander aus 1 ― 4 Aminosäuren bestehende Peptidreste darstellen,
**Z₁**, **Z₂** und **Z₃** unabhängig voneinander ―(CH₂)ₒ₁―(O―C₂H₄)ₒ₂―O―(CH₂)ₒ₃―**T** darstellen, wobei
o1 eine ganze Zahl ausgewählt aus 1, 2, 3, 4, 5 und 6 ist
o2 eine ganze Zahl ausgewählt aus 1, 2, 3, 4, 5, 6, 7, 8, 9 und 10 ist;
o3 eine ganze Zahl ausgewählt aus 1, 2, 3, 4, 5 und 6 ist;
**T** ―H, ―OCH₃, ―CO―NH₂ oder ―NH₂ darstellt; **R₁**, **R₂** und **R₃** unabhängig voneinander ausgewählt sind aus ―H, ―CH₃, ―CH₂CH₃, ―CO―und ―CO―CH₂CH₃.

11. Die Verbindung oder die pharmazeutische Zusammensetzung zur Verwendung gemäß Anspruch 10, wobei die Erkrankung ausgewählt ist aus Tumorerkrankungen, Entzündungserkrankungen, Autoimmunerkrankungen, Atemwegserkrankungen, Stoffwechselerkrankungen, Gefäßokklusionen, Herz-Kreislauf-Erkrankungen, postoperativen Zuständen und Infektionserkrankungen.

12. Die Verbindung oder die pharmazeutische Zusammensetzung zur Verwendung gemäß Anspruch 10, wobei die Erkrankung ein Herzinfarkt, Herz-Kreislauf-Stillstand, ein Schlaganfall oder eine Pankreatitis ist.

13. Die Verbindung oder die pharmazeutische Zusammensetzung zur Verwendung gemäß Anspruch 12 in Kombination mit einem Apherese- oder Dialyseverfahren zur Senkung des C-reaktives Protein-Spiegels.

14. Die pharmazeutische Zusammensetzung zur Verwendung gemäß einem der Ansprüche 10 bis 13 in Kombination mit mindestens einem Komplementblocker.

15. Die pharmazeutische Zusammensetzung zur Verwendung gemäß einem der Ansprüche 10 bis 13 in Kombination mit mindestens einem CRP bindenden Phosphocholin-Konjugat, das ein an Humanserumalbumin kovalent gebundenes 4-Aminophenylphosphorylcholin umfasst.
